# EUROPEAN PATENT APPLICATION

(11) **EP 4 810 114 A2**
(43) Date of publication of application: **23.09.2026**
(21) Application number: 26188407.6
(22) Date of filing: 01.03.2023
(51) Int. Cl.: A61P 7/04

(54) **METHODS OF ADMINISTERING FVIII MIMETIC BISPECIFIC ANTIBODIES ONCE MONTHLY**

(30) Priority: 02.03.2022 EP 22159642; 02.03.2022 EP 22159643; 08.12.2022 EP 22212144
(62) Document number(s) of the earlier application(s) in accordance with Art. 76 EPC: 23707955.3 / 4 486 792
(71) Applicant: Novo Nordisk Health Care AG, The Circle 32/38 8058 Zürich (CH)
(72) Inventor: YOFFE, Irina, 2880 Bagsværd (DK); KREILGÅRD, Mads, 2880 Bagsværd (DK)
(74) Representative: Mikkelsen, Per Jensen

(57) **Abstract**

The present invention generally relates to the use of bispecific FVIII mimetic antibodies in treatment of haemophilia such as haemophilia A with or without inhibitors and in particular methods for the treatment of the disease such as dosage regimens and compositions for use in such methods.

## Description

### TECHNICAL FIELD

The present invention relates to methods of administering Factor VIII mimetic antibodies to haemophilia patients.

### INCORPORATION-BY-REFERENCE OF THE SEQUENCE LISTING

The present application is filed with a Sequence Listing in electronic form. The entire contents of the sequence listing are hereby incorporated by reference.

### BACKGROUND

In patients with a coagulopathy, such as in human beings with haemophilia A and B, various steps of the coagulation cascade are rendered dysfunctional due to, for example, the absence or insufficient presence of a functional coagulation factor. Such dysfunction of one part of the coagulation cascade results in insufficient blood coagulation and potentially life-threatening bleeding, or damage to internal organs, such as the joints.

Coagulation Factor VIII (FVIII) deficiency, commonly referred to as haemophilia A, is a congenital bleeding disorder affecting approximately 420,000 people worldwide, of which around 105,000 are currently diagnosed.

Patients with haemophilia A may receive coagulation factor replacement therapy such as exogenous FVIII. Conventional treatment consists of replacement therapy, provided as prophylaxis or on demand treatment of bleeding episodes. Until recently prophylactic treatment for a patient with severe haemophilia A was up to three intravenous injections/week with either plasma derived FVIII or recombinant FVIII or long-acting variants thereof.

However, such patients are at risk of developing neutralizing antibodies, so-called inhibitors, to such exogenous factors, rendering formerly efficient therapy ineffective. Haemophilia A patients with inhibitors is a non-limiting example of a coagulopathy that is partly congenital and partly acquired. Patients that have developed inhibitors to FVIII cannot be treated with conventional replacement therapy.

Exogenous coagulation factors may only be administered intravenously, which is of considerable inconvenience and discomfort to patients. For example, infants and toddlers may have to have intravenous catheters surgically inserted into a chest vein, in order for venous access to be guaranteed. This leaves them at great risk of developing bacterial infections.

The drug emicizumab (HEMLIBRA^{®}) also known as ACE910, has been approved for subcutaneous prophylactic treatment of haemophilia A with or without inhibitors. Emicizumab is a humanized, bispecific anti-FIX(a)/anti-FX(a) monoclonal antibody developed by Chugai Pharmaceuticals/Roche Pharmaceuticals for the treatment of haemophilia A. Emicizumab is designed to mimic FVIII cofactor function (see Sampei et al.: (2013) PLoS One, 8, e57479 and WO2012/067176).

WO2015/194233 and WO2018/047813 disclose dosage regimens stated to be useful for administration of emicizumab.

WO2018/021450, WO2020/025672 and WO2021/152066, for example, also disclose FVIII mimetic anti-FIX(a) anti-FX(a) bispecific antibodies and their use as procoagulants for the treatment of haemophilia A.

There is a need in the art for improved methods of administering particular bispecific FVIII mimetic antibodies to patients suffering from haemophilia A, such as haemophilia A with or without inhibitors.

### SUMMARY

The present invention relates to methods of administering compounds, which serve as a substitute for coagulation Factor VIll (FVIII) in patients suffering from a coagulopathy and in particular patients lacking functional FVIII, such as haemophilia A patients including haemophilia A patients with inhibitors. In particular, bispecific antibodies capable for use in the treatment of haemophilia A with or without inhibitors, wherein said bispecific antibody is capable of binding to FIX (SEQ ID NO:1) or the activated form thereof, and FX (SEQ ID NO:2) or the activated form thereof.

In one aspect the present invention relates to a bispecific antibody for use in the treatment of haemophilia A with or without inhibitors, wherein the bispecific antibody comprises
an anti-FIX(a) antibody or antigen-binding fragment thereof capable of binding to FIX (SEQ ID NO:1) and/or the activated form thereof (FIXa) comprising a heavy chain and a light chain, and
an anti-FX(a) antibody or antigen-binding fragment thereof capable of binding to FX (SEQ ID NO:2) and/or the activated form thereof (FXa), comprising a heavy chain and a light chain,
wherein
   the heavy chain of the anti-FIX(a) antibody or antigen-binding fragment thereof comprises CDR1-3 sequences identified by SEQ ID NOs:3, 4 and 5, respectively, and
   the light chain of the anti-FIX(a) antibody or antigen-binding fragment thereof comprises CDR1-3 sequences identified by SEQ ID NOs:8, 9 and 10, respectively, and
   the heavy chain of the anti-FX(a) antibody or antigen-binding fragment thereof comprises CDR1-3 sequences identified by SEQ ID NOs:13, 14 and 15, respectively, and
   the light chain of the anti-FX(a) antibody or antigen-binding fragment thereof comprises CDR1-3 sequences identified by SEQ ID NOs:18, 19 and 20, respectively, and
   wherein the bispecific antibody is to be administered subcutaneously to a human patient in a composition comprising the bispecific antibody,
wherein a loading dose comprising
   - about 9 mg of the bispecific antibody, is administered to a patient having a body weight from 5 kg to <15, or
   - about 40 mg of the bispecific antibody, is administered to a patient having a body weight from 15 kg to <45, or
   - about 92 mg of the bispecific antibody, is administered to a patient having a body weight of 45 kg or more,
wherein a maintenance dose comprising
   - about 9 mg of the bispecific antibody, is administered once monthly to the patient having a body weight from 5 kg to <15 kg, or
   - about 20 mg of the bispecific antibody, is administered once monthly to the patient having a body weight from 15 kg to <45 kg, or
   - about 46 mg of the bispecific antibody, is administered once monthly to the patient having a body weight of 45 kg or more,
wherein the first maintenance dose is administered one month after administration of the loading dose; and
wherein a steady state plasma concentration of the bispecific antibody in the range about 2 µg/mL to about 18 µg/mL, such as 3 to 9 µg/mL, such as 6.5 µg/mL is provided.

In one embodiment said bispecific antibody comprises a first heavy chain comprising SEQ ID NO:7 and a first light chain comprising SEQ ID NO:12, and a second heavy chain comprising SEQ ID NO:17 and a second light chain comprising SEQ ID NO:22 (mAb1).

In one aspect the present invention relates to a pharmaceutical composition comprising the bispecific antibody as described herein.

In one embodiment, said bispecific antibody is administered subcutaneously to a human patient in a pharmaceutical composition comprising the bispecific antibody as described herein.

In one aspect the bispecific antibody is administered once monthly as disclosed herein.

In a preferred embodiment said antibody is administered in a loading dose followed by maintenance dosages as disclosed herein.

In another aspect the present invention relates to kits comprising a composition comprising the bispecific antibody, for example in an injection device, and instructions for use.

### BRIEF DESCRIPTION OF DRAWINGS

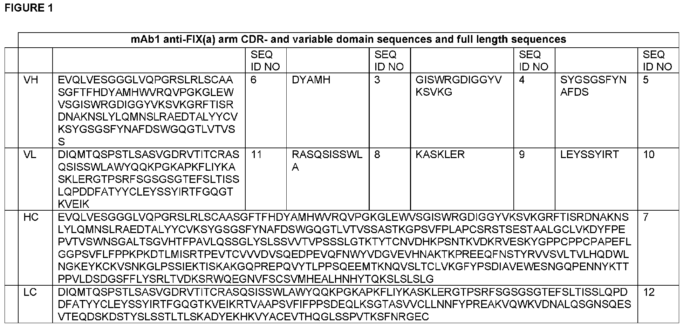

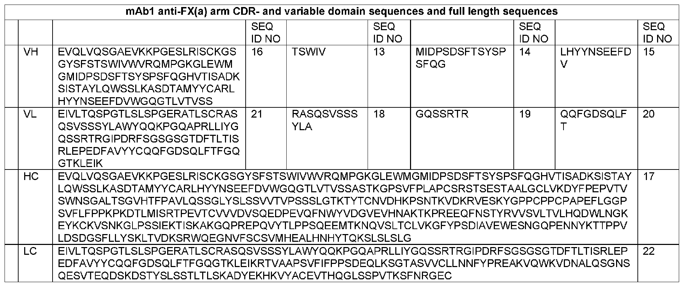

Fig. 1 presents SEQ ID NOs:3-22 in tabular format.

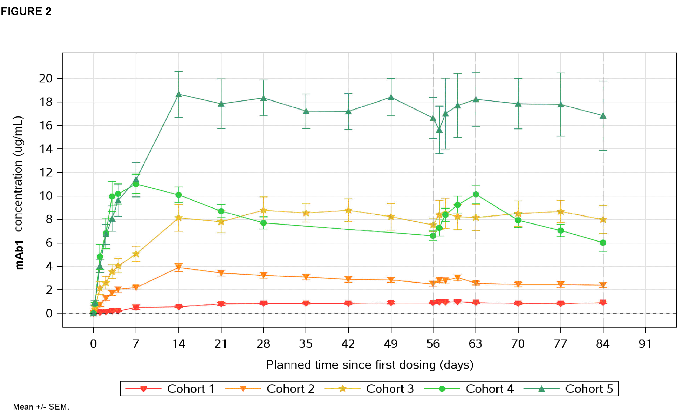

Fig. 2 shows mean profiles of mAb1 concentration in patient plasma. Pre-dose measurements below lower limit of quantitation values were set to 0. Concentration of 0 has been reported as 1e-2 g/mL due to log axis. Vertical lines indicate the two pharmacokinetics (PK) sessions. The PK session day 56-63 was used for cohorts 1-3 and 5 (once weekly) while day 56-84 was used for cohort 4 (once every 4 weeks). Mean +/- SEM.

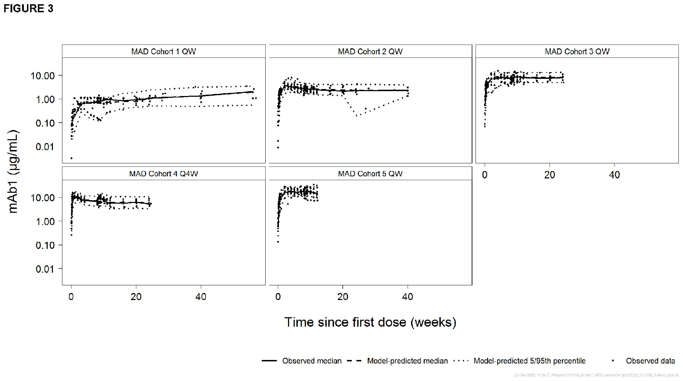

Fig. 3 shows visual predictive check of PK model fit to observed data in the FRONTIER1 MAD part. Data points are individual mAb1 plasma concentrations over time. The solid line represents median of the observed data, the dashed line represents the model-predicted median mAb1 plasma concentration versus time. The dotted lines represent the model-predicted 5th (lower) and 95th (upper) percentiles from 1000 trial simulations with the PK model. The median trendline and variability in data are adequately captured by the model across all cohorts.

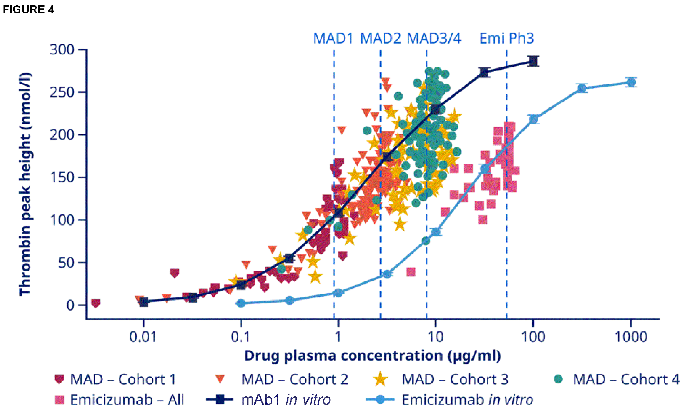

Fig. 4 shows peak thrombin levels in patients treated with increasing doses of mAb1 and a clinically recommended dose of emicizumab (emi). Plasma samples were taken from patients treated with different doses of mAb1 (Multiple Ascending Dose (MAD) cohorts), starting treatment with emicizumab or being on established prophylaxis with emicizumab at varying time points throughout the treatment period. Potential FVIII activity was neutralised by addition of anti-FVIII antibodies, and thrombin generation testing was performed *ex vivo.* The solid lines represent *in vitro* samples of human plasma from healthy subjects made haemophilia A-like with anti-FVIII antibodies, and spiked with different concentrations of mAb1 or emicizumab. The dashed line represents the mean mAb1 plasma concentration (C_{avg} was calculated based on the PK sessions) for each of the specified cohorts.

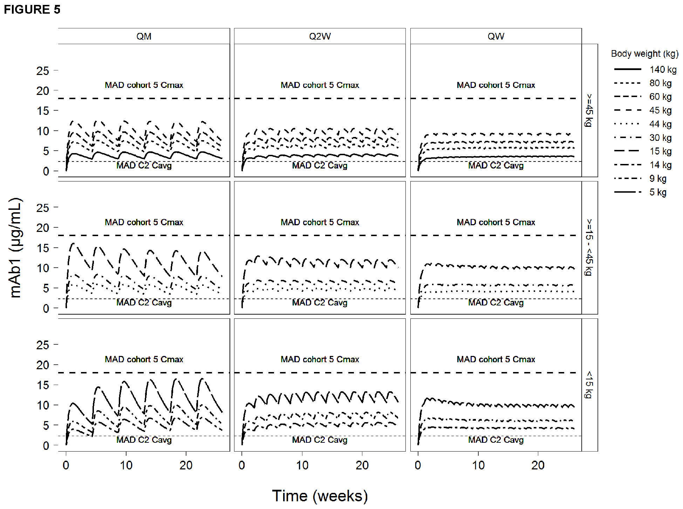

Fig. 5 shows predicted typical PK profiles for mAb1 in subjects with different body weights, using a single loading dose followed by maintenance doses as exemplified in Table 8. The PK simulations demonstrate that all subjects independent of body weight should achieve mAb1 plasma concentrations within the therapeutic range as defined by a minimum concentration comparable to FRONTIER1 MAD cohort 2 (C2) (2 µg/mL) and maximum concentration as defined by the Cₘₐₓ in MAD cohort 5 (18 µg/mL). This is demonstrated for both QW, Q2W and QM dosing intervals.

### BRIEF DESCRIPTION OF THE SEQUENCES

SEQ ID NO:1 represents the amino acid sequence of human coagulation Factor IX.
SEQ ID NO:2 represents the amino acid sequence of human coagulation Factor X.
SEQ ID NOs:3-22 represent the amino acid sequences of the components of/from the bispecific antibody "mAb1", as referred to herein, as follows:
   SEQ ID NOs:3, 4 and 5 represent Complementarity Determining Region (CDR) 1-3, respectively, of the heavy chain of the anti-FIX(a) antibody component of mAb1.
   SEQ ID NO:6 represents the heavy chain variable domain (V_{H}) of the anti-FIX(a) antibody component of mAb1.
   SEQ ID NO:7 represents the full-length heavy chain of the anti-FIX(a) antibody component of mAb1.
   SEQ ID NOs:8, 9 and 10 represent CDR 1-3, respectively, of the light chain of the anti-FIX(a) antibody component of mAb1.
   SEQ ID NO:11 represents the light chain variable domain (V_{L}) of the anti-FIX(a) antibody component of mAb1.
   SEQ ID NO:12 represents the full-length light chain of the anti-FIX(a) antibody component of mAb1.
   SEQ ID NOs:13, 14 and 15 represent CDR 1-3, respectively, of the heavy chain of the anti-FX(a) antibody component of mAb1.
   SEQ ID NO:16 represents the heavy chain variable domain (V_{H}) of the anti-FX(a) antibody component of mAb1.
   SEQ ID NO:17 represents the full-length heavy chain of the anti-FX(a) antibody component of mAb1.
   SEQ ID NOs:18, 19 and 20 represent CDR 1-3, respectively, of the light chain of the anti-FX(a) antibody component of mAb1.
   SEQ ID NO:21 represents the light chain variable domain (V_{L}) of the anti-FX(a) antibody component of mAb1.
   SEQ ID NO:22 represents the full-length light chain of the anti-FX(a) antibody component of mAb1.

Further to the electronic sequence listing as supplied herewith, Figure 1 presents SEQ ID NOs:3-22 in tabular format.

### DESCRIPTION

The present invention relates to methods of administering bispecific antibodies and compositions comprising such bispecific antibodies, which serve as a substitute for coagulation Factor VIll (FVIII) in patients suffering from a coagulopathy and in particular patients lacking functional FVIII, such as haemophilia A patients including haemophilia A patients with inhibitors and haemophilia A patients without inhibitors. In particular once monthly dosage regimens.

In order that the present invention may be more readily understood, certain terms are first defined.

The term "a" or "an" is intended to mean "one or more." The term "comprise" and variations thereof such as "comprises" and "comprising," when preceding the recitation of a step or an element, are intended to mean that the addition of further steps or elements is optional and not excluded.

The term "about" is used herein to mean approximately, roughly or around. When the term "about" is used in conjunction with a numerical range, it modifies that range by extending the boundaries above and below the numerical values set forth. In general, the term "about" can modify a numerical value above and below the stated value by 10 percent, up or down (higher or lower).

The term "annualized bleeding rate" (ABR) refers to the number of treated bleeding episodes (including spontaneous and traumatic bleeds) experienced by a patient during a defined time period, extrapolated to one year. For example, two bleeds in six months would indicate an ABR of four.

The term "antibody" includes - but is not limited to - antibodies that are bivalent, such as bispecific antibodies. Full-length antibodies comprise at least four polypeptide chains: two heavy chains (HC) and two light chains (LC) that are connected by disulfide bonds. One class of immunoglobulins of particular pharmaceutical interest is the IgGs. In humans, the IgG class may be divided into four sub-classes IgG1, IgG2, IgG3 and in a preferred embodiment IgG4, based on the sequence of their heavy chain constant regions. The light chains can be divided into two types, kappa and lambda chains, based on differences in their sequence composition. IgG molecules are composed of two heavy chains, interlinked by two or more disulfide bonds, and two light chains, each attached to a heavy chain by a disulfide bond. An IgG heavy chain may comprise a heavy chain variable domain (V_{H}) and up to three heavy chain constant (C_{H}) domains: C_{H}1, C_{H}2 and C_{H}3. A light chain may comprise a light chain variable domain (V_{L}) and a light chain constant domain (C_{L}). V_{H} and V_{L} regions can be further subdivided into regions of hypervariability, termed complementarity determining regions (CDRs) or hypervariable regions (HvRs), interspersed with regions that are more conserved, termed framework regions (FR). V_{H} and V_{L} domains are typically composed of three CDRs and four FRs, arranged from amino-terminus to carboxy-terminus in the following order: FR1, CDR1, FR2, CDR2, FR3, CDR3, FR4. The heavy and light chain variable domains containing the hypervariable regions (CDRs) form a structure that is capable of interacting with an antigen, whilst the constant region of an antibody may mediate binding of the immunoglobulin to host tissues or factors, including, but not limited to various cells of the immune system (effector cells), Fc receptors and the first component, C1q, of the C1 complex of the classical complement system.

Antibodies or fragment thereof may be defined in terms of their complementarity-determining regions (CDRs). The term "complementarity-determining region" or "CDR", when used herein, refers to the regions of an antibody in which amino acid residues involved in antigen-binding are situated. The CDRs can be identified as the regions with the highest variability in amino acid alignments of antibody variable domains. Databases can be used for CDR identification such as the Kabat database, the CDRs e.g. being defined as comprising amino acid residues 24-34 (L1), 50-56 (L2) and 89-97 (L3) of the light-chain variable domain and 31-35 (H1), 50-65 (H2) and 95-102 (H3) in the heavy-chain variable domain; (Kabat et al. 1991; Sequences of Proteins of Immunological Interest, Fifth Edition, U.S. Department of Health and Human Services, NIH Publication No. 91-3242). Typically, the numbering of amino acid residues in this region is performed by the method described in Kabat *et al. supra*. Phrases such as "Kabat position", "Kabat residue", and "according to Kabat" herein refer to this numbering system for heavy chain variable domains or light chain variable domains, and unless contradicted by the context numbering according to Kabat is used herein.

The term "bispecific antibody" as used herein, refers to an antibody which is capable of binding to two different antigens or two different epitopes on the same antigen.

The term "fixed dose" of the bispecific antibody, refers to a dose that is administered to a patient having a body weight falling within a predetermined range (such as 15 kg to <45 kg). The fixed dose is therefore not provided as a mg/kg dose, but rather as an absolute amount of the bispecific antibody.

The term "human antibody", as used herein, is intended to include antibodies having variable domains in which at least a portion of a framework region and/or at least a portion of a CDR region are derived from human germline immunoglobulin sequences. For example, a human antibody may have variable domains in which both the framework and CDR regions are derived from human germline immunoglobulin sequences. Furthermore, if the antibody contains a constant region, the constant region or a portion thereof is also derived from human germline immunoglobulin sequences. Preferably, a human antibody is monoclonal antibody.

As used herein "mAb1" refers to a bispecific antibody comprising an anti-FIX(a) antibody capable of binding to FIX (SEQ ID NO:1) and/or the activated form thereof (FIXa) comprising a heavy chain and a light chain, and an anti-FX(a) antibody capable of binding to FX (SEQ ID NO:2) and/or the activated form thereof (FXa), comprising a heavy chain and a light chain, wherein the heavy chain of the anti-FIX(a) antibody comprises SEQ ID NO:7, the light chain of the anti-FIX(a) antibody comprises SEQ ID NO:12, the heavy chain of the anti-FX(a) antibody comprises SEQ ID NO:17 and the light chain of the anti-FX(a) antibody comprises SEQ ID NO:22. The bispecific antibody comprises CDR sequences represented by SEQ ID NOs:3, 4, 5 and 8, 9, 10, and 13, 14, 15 and 18, 19, 20.

The term "dosage regimen" or "dosing regimen" includes a treatment regimen based on a determined set of doses. For example, in one embodiment, the invention describes dosage regimens for the treatment of haemophilia A with or without inhibitors wherein the bispecific antibody is first administered in a loading dose and then administered in maintenance doses comprising the same or a lower amount of the bispecific antibody than that of the loading dose.

The term "dosing" refers to the administration of a substance (such as mAb1) to achieve a therapeutic objective (e.g., the treatment of haemophilia A with or without inhibitors).

A "dose" can be administered in a single administration or in multiple successive administrations. For example, a 60 mg dose can be administered either in a single 60 mg administration or in two successive administrations of 30 mg each, and a 120 mg dose can, for example, be administered in three successive administrations of 40 mg. Successive administration of doses within 1 hour of administration of the first dose in sum constitute one dose (for example a loading dose which cannot readily be administered in a single administration).

The term "FIX (SEQ ID NO:1) and/or the activated form thereof (FIXa)" may also be referred to as "FIX/FIXa" or simply "FIX(a)".

The term "FX (SEQ ID NO:2) and/or the activated form thereof (FXa)" may also be referred to as "FX/FXa" or simply "FX(a)".

The term "heavy chain" includes a full-length heavy chain. A full-length heavy chain includes a variable region domain, V_{H}, and three constant region domains, C_{H}1, C_{H}2, and C_{H}3. The V_{H} domain is at the amino-terminus of the polypeptide, and the C_{H} domains are at the carboxyl-terminus, with the C_{H}3 being closest to the -COOH end.

The term "light chain" as used herein includes a full-length light chain. A full-length light chain includes a variable region domain, V_{L}, and a constant region domain, C_{L}. The variable region domain of the light chain is at the amino-terminus of the polypeptide. Light chains as described herein include kappa chains and lambda chains.

The term "kit" refers to a packaged product comprising components with which to administer the bispecific antibody (such as mAb1) for treatment of a disorder and instructions for use. The kit preferably comprises a box or container that holds the components of the kit. The box or container is affixed with a label or a Food and Drug Administration (or corresponding Authority) approved protocol.

The term "loading dose" as used herein refers to a first dose of the bispecific antibody administered to the patient at the start of the treatment regimen. In general, the loading dose is intended to achieve a therapeutically relevant plasma concentration of the bispecific antibody in the body of the patient within a short timeframe.

The term "loading period" refers to a period of treatment of a patient comprising administration of the bispecific antibody to the patient in order to induce a clinical response. The "loading period" is typically between one week and one month in duration depending on the desired frequency of administration and is triggered by administration of a first loading dose. The loading period precedes administration of the first maintenance dose.

The term "maintenance dose" as used herein relates to a dose of the bispecific antibody administered to the patient at a point in time after administration of the loading dose.

The term "maximum plasma concentration" (Cₘₐₓ) means the highest observed concentration of a bispecific antibody in patient plasma after administration of the bispecific antibody to the patient.

The term "average plasma concentration" (or "C_{avg}") refers to the average plasma concentration of the bispecific antibody within a dosing interval at steady state.

The term "steady state Cₘₐₓ of mAb1 plasma concentration" refers to the state, wherein the post dose maximum plasma concentration of mAb1 does not differ from one dose to another. In one embodiment, a steady state Cₘₐₓ of the mAb1 plasma concentration is about 18 µg/mL. In another embodiment, a steady state Cₘₐₓ of the mAb1 plasma concentration is about 9 µg/mL.

The term "steady state Cₘᵢₙ of mAb1 plasma concentration" refers to the state, wherein the post-dose minimum plasma concentration of mAb1 does not differ from one dose to another. In one embodiment, a steady state Cₘᵢₙ of the mAb1 plasma concentration is about 2 µg/mL. In another embodiment, a steady state Cₘᵢₙ of the mAb1 plasma concentration is about 3 µg/mL.

The term "serum or plasma half-life" refers to the time required for half the quantity of a substance administered to a patient to be metabolized or eliminated from the serum or plasma of the patient by normal biological processes.

The term "prophylactic treatment" refers to the administration of a therapy for the treatment of haemophilia A with or without inhibitors, where such treatment is intended to control, manage, prevent or reduce the occurrence and/or severity of one or more symptoms of for example haemophilia A with or without inhibitors, e.g., bleeding episodes, e.g., one or more spontaneous bleeding episodes, and/or joint damage.

The terms "treatment" or "treating" means reduction of the frequency of one or more symptoms of haemophilia A with or without inhibitors, e.g., spontaneous or uncontrollable bleeding episodes. "Treatment", however, need not be a cure.

The term "Tₘₐₓ" refers to the observed time for reaching the maximum concentration of a substance in plasma of a patient after administration of that substance to the patient.

The present invention relates to methods of administering bispecific antibodies, which serve as a substitute for coagulation Factor VIll (FVIII) in patients suffering from a coagulopathy and in particular patients lacking functional FVIII, such as haemophilia A patients including haemophilia A patients with inhibitors and haemophilia A patients without inhibitors. In particular, bispecific antibodies or an antigen-binding fragment thereof capable for use in the treatment of haemophilia A with or without inhibitors, wherein said antibody is capable of binding to FIX (SEQ ID NO:1) or the activated form thereof, and FX (SEQ ID NO:2) or the activated form thereof.

In one such embodiment the heavy chain of the anti-FIX(a) antibody or antigen-binding fragment thereof comprises CDR1-3 sequences identified by SEQ ID NOs:3, 4 and 5 respectively, and the light chain of the anti-FIX(a) antibody or antigen-binding fragment thereof comprises CDR1-3 sequences identified by SEQ ID NOs:8, 9 and 10, respectively, and the heavy chain of the anti-FX(a) antibody or antigen-binding fragment thereof comprises CDR1-3 sequences identified by SEQ ID NOs:13, 14 and 15, respectively, and the light chain of the anti-FX(a) antibody or antigen-binding fragment thereof comprises CDR1-3 sequences are identified by SEQ ID NOs:18, 19 and 20, respectively.

In one such embodiment the anti-FIX(a) antibody or antigen-binding fragment thereof comprises a heavy chain variable domain identified by SEQ ID NO:6 and a light chain variable domain identified by SEQ ID NO:11, and the anti-FX(a) antibody or antigen-binding fragment thereof comprises a heavy chain variable domain identified by SEQ ID NO:16 and a light chain variable domain identified by SEQ ID NO:21.

In a preferred embodiment, the bispecific antibody is of the IgG4 isotype.

In a preferred embodiment, the bispecific antibody is a human antibody.

In one embodiment, the heavy chain of the anti-FIX(a) antibody comprises SEQ ID NO:7 and the light chain of the anti-FIX(a) antibody comprises SEQ ID NO:12, and the heavy chain of the anti-FX(a) antibody comprises SEQ ID NO:17 and the light chain of the anti-FX(a) antibody comprises SEQ ID NO:22 (also referred to herein as mAb1).

The properties of the bispecific antibody have been described in WO2020/025672, which is incorporated by reference herein.

As is apparent from the above the bispecific antibody can thus be characterised with reference to its CDR sequences, its variable domain sequences or its full heavy and light chain sequences. For avoidance of doubt, reference to mAb1 entails the presence of the heavy chains and light chains as defined by SEQ ID NOs:7 and 12, and 17 and 22 herein. The term "mAb1" is used interchangeably with the term "bimAb1".

The methods disclosed herein include administering once monthly, a subcutaneous injections of the bispecific antibody to the patient. Preferably, administration of one or more loading dose(s) precede the once monthly administration scheme.

Accordingly, in a first aspect of the present invention, there is provided a method of treating haemophilia A with or without inhibitors comprising administering to a patient in need thereof an effective amount of a bispecific antibody, said method comprising
a) administering at least one loading dose of the bispecific antibody to the patient;
   and
b) administering at least one maintenance dose(s) of the bispecific antibody to the patient after the last loading dose is administered.

In one aspect, such bispecific antibody is mAb1.

In a preferred embodiment, the loading dose and maintenance dose is selected based on the body weight of the patient and in particular patient body weight ranges (also referred to herein as "weight bands").

In such embodiment, patients having a body weight of from 5 kg to <15 kg are grouped.

In another such embodiment, patients having a body weight of from 15 kg to <45 kg are grouped.

In yet another such embodiment, patients having a body weight of 45 kg or more are grouped.

In one embodiment, the loading dose(s) and maintenance doses, respectively, are fixed doses suitable for use in patients having a body weight from 5 kg to <15 kg.

In one embodiment, the loading dose(s) and maintenance doses, respectively, are fixed doses suitable for use in patients having a body weight from 15 kg to <45 kg.

In one embodiment, the loading dose(s) and maintenance doses, respectively, are fixed doses suitable for use in patients having a body weight of 45 kg or more.

In one embodiment the loading dose(s) and maintenance dose(s) as measured in mg is/are identical in terms of the amount of bispecific antibody to be delivered.

In one embodiment the loading dose(s) and maintenance dose(s) as measured in mg is/are not identical in terms of the amount of bispecific antibody to be delivered.

The method of administration as disclosed herein include once monthly dosage regimens, such regimens factor in particular patient body weight bands and dosages carefully designed by the inventors such to allow for safe and efficacious treatment.

Due to a long half-life of mAb1, coupled with limited between-subject variability in plasma concentration, the large therapeutic window and dosing near maximal effect as determined by the inventors, it is considered feasible to dose using simplified weight bands instead of continuous body-weight-based dosing. As well as offering safe and effective haemostatic coverage, the weight band-based dosing with a fixed volume injection will also be more convenient than dosing per kg body weight, requiring no dose calculation and reducing risk of medication errors. It aims to simplify administration while still accounting for weight- and drug product strength related differences in plasma concentration (see example 1, table 6). This dosing modality is suitable for injection devices such as - but not limited to - a pre-filled pen-injector for subcutaneous administration of mAb1.

### Once monthly dosage regimen ranges

In preferred embodiments, a loading dose(s) comprising about 5 mg to about 15 mg, such as 9 mg, of a bispecific antibody, such as mAb1, is administered to patients having a body weight of from 5 kg to <15 kg, and a loading dose(s) comprising about 35 mg to about 45 mg, such as 40 mg, of the bispecific antibody, such as mAb1, is administered to patients having a body weight of from 15 kg to <45 kg, and a loading dose(s) comprising about 85 mg to about 95 mg, such as 92 mg, of the bispecific antibody, such as mAb1, is administered to patients having a body weight of 45 kg or more.

In preferred embodiments, maintenance dose(s) comprising about 7 mg to about 11 mg, such as 9 mg, of a bispecific antibody, such as mAb1, is administered to patients having a body weight of from 5 kg to <15 kg, and maintenance dose(s) comprising about 18 mg to about 22 mg, such as 20 mg, of a bispecific antibody, such as mAb1, is administered to patients having a body weight of from 15 kg to <45 kg, and maintenance dose(s) comprising about 44 mg to about 48 mg, such as 46 mg, of the bispecific antibody, such as mAb1, is administered to patients having a body weight of 45 kg or more.

In a preferred embodiment the first maintenance dose is administered one month after administration of the loading dose.

In preferred embodiments, administration of once monthly maintenance doses will continue for as long as treatment is necessary.

In one embodiment, methods are provided for the treatment of haemophilia, such as haemophilia A with or without inhibitors, wherein the method comprises administering to said patient a composition comprising a bispecific antibody capable of binding to FIX(a) and FX(a), wherein said administration provides a steady state plasma concentration of said bispecific antibody in the range 2 µg/mL to about 18 µg/mL, preferably in the range about 3 µg/mL to about 9 µg/mL, such as 6 to 7 µg/mL, such as 6.5 to 7 µg/mL.

In a preferred embodiment, the bispecific antibody comprises an anti-FIX(a) antibody or antigen-binding fragment thereof capable of binding to FIX (SEQ ID NO:1) and/or the activated form thereof (FIXa) comprising a heavy chain and a light chain, and an anti-FX(a) antibody or antigen-binding fragment thereof capable of binding to FX (SEQ ID NO:2) and/or the activated form thereof (FXa), comprising a heavy chain and a light chain, wherein the heavy chain of the anti-FIX(a) antibody or antigen-binding fragment thereof comprises CDR1-3 sequences identified by SEQ ID NOs:3, 4 and 5 respectively, and the light chain of the anti-FIX(a) antibody or antigen-binding fragment thereof comprises CDR1-3 sequences identified by SEQ ID NOs:8, 9 and 10, respectively, and the heavy chain of the anti-FX(a) antibody or antigen-binding fragment thereof comprises CDR1-3 sequences identified by SEQ ID NOs:13, 14 and 15, respectively, and the light chain of the anti-FX(a) antibody or antigen-binding fragment thereof comprises CDR1-3 sequences are identified by SEQ ID NOs:18, 19 and 20, respectively.

In a more preferred embodiment, the anti-FIX(a) antibody or antigen-binding fragment thereof comprises a heavy chain variable domain identified by SEQ ID NO:6 and a light chain variable domain identified by SEQ ID NO:11, and the anti-FX(a) antibody or antigen-binding fragment thereof comprises a heavy chain variable domain identified by SEQ ID NO:16 and a light chain variable domain identified by SEQ ID NO:21.

In a most preferred embodiment, the heavy chain of the anti-FIX(a) antibody comprises SEQ ID NO:7 and the light chain of the anti-FIX(a) antibody comprises SEQ ID NO:12, and the heavy chain of the anti-FX(a) antibody comprises SEQ ID NO:17 and the light chain of the anti-FX(a) antibody comprises SEQ ID NO:22.

### Loading doses in once monthly dosage regimens for mAb1 as characterized by its heavy- and light chain CDR sequences or heavy- and light chain variable domain sequences or full-length heavy - and light chain sequences

In one embodiment, the composition comprising the bispecific antibody is administered subcutaneously as a loading dose in a once monthly dosage regimen, which may be at a dose of 8.5, 8.6, 8.7, 8.8, 8.9, 9, 9.1, 9.2, 9.3, 9.4 or 9.5 mg, preferably 9 mg, in patients having a body weight of 5 kg to <15 kg.

In one embodiment, the composition comprising the bispecific antibody is administered subcutaneously as a loading dose in a once monthly dosage regimen, which may be at a dose of 39.5, 39.6, 39.7, 39.8, 39.9, 40, 40.1, 40.2, 40.3, 40.4 or 40.5 mg, preferably 40 mg, in patients having a body weight of 15 kg to <45 kg.

In one embodiment, the composition comprising the bispecific antibody is administered subcutaneously as a loading dose in a once monthly dosage regimen, which may be at a dose of 91.5, 91.6, 91.7, 91.8, 91.9, 92, 92.1, 92.2, 92.3, 92.4 or 92.5 mg, preferably 92 mg, in patients having a body weight of 45 kg or more.

### Maintenance doses in once monthly dosage regimens for mAb1 as characterized by its heavy- and light chain CDR sequences or heavy- and light chain variable domain sequences or full-length heavy - and light chain sequences

In one embodiment, the composition comprising the bispecific antibody is administered subcutaneously as a maintenance dose in a once monthly dosage regimen, which may be at a dose of 8.5, 8.6, 8.7, 8.8, 8.9, 9, 9.1, 9.2, 9.3, 9.4 or 9.5 mg, preferably 9 mg, in patients having a body weight of 5 kg to <15 kg.

In one embodiment, the composition comprising the bispecific antibody is administered subcutaneously as a maintenance dose in a once monthly dosage regimen, which may be at a dose of 19.5, 19.6, 19.7, 19.8, 19.9, 20, 20.1, 20.2, 20.3, 20.4 or 20.5 mg, preferably 20 mg, in patients having a body weight of 15 kg to <45 kg.

In one embodiment, the composition comprising the bispecific antibody is administered subcutaneously as a maintenance dose in a once monthly dosage regimen, which may be at a dose of 45.5, 45.6, 45.7, 45.8, 45.9, 46, 46.1, 46.2, 46.3, 46.4 or 46.5 mg, preferably 46 mg, in patients having a body weight of 45 kg or more.

In one embodiment, the methods of administration as disclosed herein provide an ABR of 1, 2, 3, 4 or 5 or in the range 0-3 such as 1-3 or 2-3 or in the range 1-5, such as 1-2, 1-3, 1-4, 2-3, 2-4, 2-5, 3-4, 3-5 or 4-5..

In one embodiment, the method of administration comprises administration of one, two or three further loading doses - termed "extended loading dose" to distinguish it from the initial loading dose - if the patient does not achieve an appropriate clinical response at the end of the initial loading period. The dose and dosing intervals during the extended loading period are typically the same as dose and dosing intervals during the initial loading period, but may be changed if the attending health care professional has reason to believe that the patient may benefit from changes such as an increased dose of the bispecific antibody or more frequent dosing.

In one embodiment, the first maintenance dose is administered one month after the loading dose is administered to the patient.

In one embodiment, T_{1/2} is about 30.4 days.

In one embodiment, Tₘₐₓ is about 9.1 days.

In a preferred embodiment, the treatment as disclosed herein is a prophylactic treatment.

In one embodiment, the methods of administration as disclosed herein reduces spontaneous bleeding or bleeding episodes in a patient susceptible to such spontaneous bleeding or bleeding episodes.

### Pharmaceutical formulations

In one aspect of the invention a pharmaceutical composition is provided which is suitable for use with the methods of administration disclosed herein.

Such pharmaceutical composition comprises a bispecific antibody, which is preferably present in a concentration from 1 mg/mL to 100 mg/mL, such as 2 mg/mL to 100 mg/mL, such as 2 mg/mL to 60 mg/mL, and has a pH in the range 5.5 to 7.5, preferably in the range 6.0-6.5, such as about 6.3, such as 6.3.

In more preferred embodiments, the concentration of the bispecific antibody is 2 mg/mL, 5 mg/mL, 11.25 mg/mL, 25 mg/mL or 57.5 mg/mL.

Such pharmaceutical composition are suitable for use in - but not limited to - fixed-dose injection devices, for example injection devices configured to administer 0.8 ml per injection.

In a preferred embodiment, the pharmaceutical composition is an aqueous formulation. In one embodiment, the bispecific antibody is mAb1.

The pharmaceutical composition may further comprise one or more of: a buffer system, a preservative, a tonicity agent, a chelating agent, a stabilizer, or a surfactant, as well as various combinations thereof. The use of preservatives, isotonic agents, chelating agents, stabilizers and surfactants in pharmaceutical compositions is well-known to the skilled person. Reference may be made to Remington: The Science and Practice of Pharmacy, 19^{th} edition, 1995.

In one embodiment, the pharmaceutical composition comprises 1 mg/mL to 100 mg/mL of a bispecific antibody such as mAb1, L-arginine or L-arginine hydrochloride, L-histidine and a surfactant at a pH in the range 5.5 to 7.0.

In one embodiment, the pharmaceutical composition comprises 1 mg/mL to 100 mg/mL of a bispecific antibody such as mAb1, about 150 mM of L-arginine hydrochloride, about 20 mM of L-histidine, polysorbate 20 or polysorbate 80 at a pH in the range 5.5-7.0.

In one embodiment, the pharmaceutical composition comprises 2 mg/mL, 5 mg/mL, 11.25 mg/mL, 25 mg/mL or 57.5 mg/mL of the bispecific antibody mAb1, about 150 mM of L-arginine hydrochloride, about 20 mM of L-histidine, about 0.02% polysorbate 20 at about pH 6.3.

In preferred embodiments, the pharmaceutical composition comprises about 2 mg/mL, about 5 mg/mL, about 11.25 mg/mL, about 25 mg/mL or about 57.5 mg/mL of the bispecific antibody mAb1, about 150 mM of L-arginine hydrochloride, about 20 mM of L-histidine, about 0.02% polysorbate 20 at about pH 6.3.

In a preferred embodiment, the pharmaceutical composition comprises 2 mg/mL of the bispecific antibody mAb1, 150 mM of L-arginine hydrochloride, 20 mM of L-histidine, 0.02% polysorbate 20 at pH 6.3.

In another preferred embodiment, the pharmaceutical composition comprises 5 mg/mL of the bispecific antibody mAb1, 150 mM of L-arginine hydrochloride, 20 mM of L-histidine, 0.02% polysorbate 20 at pH 6.3.

In another preferred embodiment, the pharmaceutical composition comprises 11.25 mg/mL of the bispecific antibody mAb1, 150 mM of L-arginine hydrochloride, 20 mM of L-histidine, 0.02% polysorbate 20 at pH 6.3.

In another preferred embodiment, the pharmaceutical composition comprises 25 mg/mL of the bispecific antibody mAb1, 150 mM of L-arginine hydrochloride, 20 mM of L-histidine, 0.02% polysorbate 20 at pH 6.3.

In yet another preferred embodiment, the pharmaceutical composition comprises 57.5 mg/mL of the bispecific antibody mAb1, 150 mM of L-arginine hydrochloride, 20 mM of L-histidine, 0.02% polysorbate 20 at pH 6.3.

In one embodiment, the pharmaceutical composition comprises 2 mg/mL, 5 mg/mL, 11.25 mg/mL, 25 mg/mL or 57.5 mg/mL of the bispecific antibody mAb1, about 150 mM of L-arginine hydrochloride, about 20 mM of L-histidine, about 0.02 w/v% polysorbate 20 at about pH 6.3.

In preferred embodiments, the pharmaceutical composition comprises about 2 mg/mL, about 5 mg/mL, about 11.25 mg/mL, about 25 mg/mL or about 57.5 mg/mL of the bispecific antibody mAb1, about 150 mM of L-arginine hydrochloride, about 20 mM of L-histidine, about 0.02 w/v% polysorbate 20 at about pH 6.3.

In a preferred embodiment, the pharmaceutical composition comprises 2 mg/mL of the bispecific antibody mAb1, 150 mM of L-arginine hydrochloride, 20 mM of L-histidine, 0.02 w/v% polysorbate 20 at pH 6.3.

In another preferred embodiment, the pharmaceutical composition comprises 5 mg/mL of the bispecific antibody mAb1, 150 mM of L-arginine hydrochloride, 20 mM of L-histidine, 0.02 w/v% polysorbate 20 at pH 6.3.

In another preferred embodiment, the pharmaceutical composition comprises 11.25 mg/mL of the bispecific antibody mAb1, 150 mM of L-arginine hydrochloride, 20 mM of L-histidine, 0.02 w/v% polysorbate 20 at pH 6.3.

In another preferred embodiment, the pharmaceutical composition comprises 25 mg/mL of the bispecific antibody mAb1, 150 mM of L-arginine hydrochloride, 20 mM of L-histidine, 0.02 w/v% polysorbate 20 at pH 6.3.

In yet another preferred embodiment, the pharmaceutical composition comprises 57.5 mg/mL of the bispecific antibody mAb1, 150 mM of L-arginine hydrochloride, 20 mM of L-histidine, 0.02 w/v% polysorbate 20 at pH 6.3.

In one embodiment, a pharmaceutical composition comprising a mAb1 concentration of 2 mg/mL is used to administer a dose of 1.6 mg of mAb1.

In one embodiment, a pharmaceutical composition comprising a mAb1 concentration of 5 mg/mL is used to administer a dose of 4 mg of mAb1.

In one embodiment, a pharmaceutical composition comprising a mAb1 concentration of 11.25 mg/mL is used to administer a dose of 9 mg of mAb1.

In one embodiment, a pharmaceutical composition comprising a mAb1 concentration of 25 mg/mL is used to administer a dose of 20 mg of mAb1.

In one embodiment, a pharmaceutical composition comprising a mAb1 concentration of 57.5 mg/mL is used to administer a dose of 46 mg of mAb1.

In some embodiments, the pharmaceutical composition as disclosed herein is intended for use and/or contained in an injection device.

In a preferred embodiment, the injection device is a fixed dose device, such as one configured to deliver a single or a device configured to deliver multiple predetermined doses of the pharmaceutical composition, the latter sometimes being referred to as a multiple fixed dose device or a fixed dose, multi-shot device. In some embodiments, the injection device is a disposable, pre-filled, multi-dose device. In some embodiments, the injection device is a disposable, pre-filled, single shot device.

In one embodiment, the pharmaceutical composition of the invention is administered using an injection device comprising a tube having a needle gauge in the range from 26 to 36.

In one embodiment, the loading dose and/or maintenance dose may be administered as single injection(s), respectively, wherein the entire loading dose and/or maintenance dose is administered as a single administration, i.e. wherein the entire dose is administered all at once.

In some embodiments, the loading dose and/or maintenance dose is administered in multiple smaller doses, for example in 2, 3 or 4 smaller doses in sum making up the full loading- or maintenance dose. As a non-limiting example, a loading dose of 80 mg of bispecific antibody can be administered in three smaller doses of 60 mg each. Alternatively, two 50 mg doses and one 80 mg dose administered consecutively could - for example - also be contemplated.

The pharmaceutical composition can be administered as a subcutaneous injection of at least 0.05 mL injection solution, such to arrive at a desired dose as measured in mg.

By way of example, for a pharmaceutical composition comprising 100 mg/mL of compound of mAb1, a volume of 100 µL is required to provide a dose of 10 mg.

The necessary volume will depend on the concentration of bispecific antibody in the pharmaceutical composition to be administered, since a lower volume typically would make additional injections necessary and a higher volume typically would lead to discomfort for the patient at the injection site. Typically, a volume of between 0.08 and 1.5 mL injection solution, preferably between 0.2 and 1 mL, more preferably between 0.6 and 0.9 mL, more preferably 0.8 mL, is administered per injection.

The pharmaceutical compositions described above can be combined such to allow for particular doses - as disclosed herein - to be administered using a (fixed) injection solution volume of 0.8 mL per injection. For example, two injections of 0.8 mL of the pharmaceutical composition comprising 57.5 mg/mL mAb1 will allow for a cumulative dose of 92 mg of mAb1. In another example, one injection of 0.8 mL of the pharmaceutical composition comprising 11.25 mg followed by one injection of 0.8 mL of the pharmaceutical composition comprising 57.5 mL will allow for a cumulative dose of 55 mg of mAb1.

The pharmaceutical composition may be administered at the same or different injection sites.

In another general aspect, the invention pertains to a kit comprising an injection device containing a pharmaceutical composition comprising mAb1 and one or more pharmaceutically acceptable carrier(s). The kit contains instructions for subcutaneous dosing of the pharmaceutical composition for the treatment of haemophilia A with or without inhibitors.

### Further embodiments

1. A dosage regimen for use in the treatment of haemophilia A with or without inhibitors, comprising subcutaneously administering a bispecific antibody comprising an anti-FIX(a) antibody or antigen-binding fragment thereof capable of binding to FIX (SEQ ID NO:1) and/or the activated form thereof (FIXa) comprising a heavy chain and a light chain, and an anti-FX(a) antibody or antigen-binding fragment thereof capable of binding to FX (SEQ ID NO:2) and/or the activated form thereof (FXa), comprising a heavy chain and a light chain,
   wherein the heavy chain of the anti-FIX(a) antibody or antigen-binding fragment thereof comprises CDR1-3 sequences identified by SEQ ID NOs:3, 4 and 5 respectively, and
   the light chain of the anti-FIX(a) antibody or antigen-binding fragment thereof comprises CDR1-3 sequences identified by SEQ ID NOs:8, 9 and 10, respectively, and
   the heavy chain of the anti-FX(a) antibody or antigen-binding fragment thereof comprises CDR1-3 sequences identified by SEQ ID NOs:13, 14 and 15, respectively, and
   the light chain of the anti-FX(a) antibody or antigen-binding fragment thereof comprises CDR1-3 sequences are identified by SEQ ID NOs:18, 19 and 20, respectively;
   in one or more loading dose followed by administration of once monthly maintenance doses,
   wherein the loading dose comprises the same or a higher amount of the bispecific antibody than the maintenance dose.
2. The dosage regimen according to embodiment 1 wherein the anti-FIX(a) antibody or antigen-binding fragment thereof comprises
   a heavy chain variable domain identified by SEQ ID NO:6 and a light chain variable domain identified by SEQ ID NO:11, and
   the anti-FX(a) antibody or antigen-binding fragment thereof comprises a heavy chain variable domain identified by SEQ ID NO:16 and a light chain variable domain identified by SEQ ID NO:21.
3. The dosage regimen according to embodiment 1 or 2 wherein
   the heavy chain of the anti-FIX(a) antibody comprises SEQ ID NO:7 and the light chain of the anti-FIX(a) antibody comprises SEQ ID NO:12, and
   the heavy chain of the anti-FX(a) antibody comprises SEQ ID NO:17 and the light chain of the anti-FX(a) antibody comprises SEQ ID NO:22.
4. The dosage regimen according to any of the former embodiments, wherein the bispecific antibody is administered in a pharmaceutical composition comprising the bispecific antibody.
5. The dosage regimen according to any of the former embodiments, wherein the pharmaceutical composition comprises 1 mg/mL to 100 mg/mL of the bispecific antibody, L-arginine or L-arginine hydrochloride, L-histidine and a surfactant at about pH 5.5 to about pH 7.
6. The dosage regimen according to any of the former embodiments, wherein the pharmaceutical composition comprises 1 mg/mL to 100 mg/mL of the bispecific antibody, L-arginine or L-arginine hydrochloride, L-histidine and a surfactant at about pH 6.3.
7. The dosage regimen according to any of the former embodiments, wherein the pharmaceutical composition comprises 1 mg/mL to 100 mg/mL of the bispecific antibody, about 150 mM of L-arginine, about 20 mM of L-histidine and about 0.02% polysorbate 20 or 80 at about pH 6.3.
8. The dosage regimen according to any of the former embodiments, wherein the pharmaceutical composition comprises 1 mg/mL to 100 mg/mL of the bispecific antibody, about 150 mM of L-arginine hydrochloride, about 20 mM of L-histidine and about 0.02% polysorbate 20 at about pH 6.3.
9. The dosage regimen according to any of the former embodiments, wherein the pharmaceutical composition comprises 2 mg/mL to 60 mg/mL of mAb1, such as 2 mg/mL, 5 mg/mL, 11.25 mg/mL, 25 mg/mL or 57.5 mg/mL of mAb1, 150 mM of L-arginine hydrochloride, 20 mM of L-histidine and 0.02% polysorbate 20 at pH 6.3.
10. The dosage regimen according to any of the former embodiments, wherein the bispecific antibody is administered to a human patient.
11. The dosage regimen according to any of embodiments 1-10 wherein a loading dose comprising
   about 5 mg to about 15 mg of the bispecific antibody, is administered to a patient having a body weight from 5 kg to <13, or
   about 35 mg to about 45 mg of the bispecific antibody, is administered to a patient having a body weight from 15 kg to <45, or
   about 85 mg to about 95 mg of the bispecific antibody, is administered to a patient having a body weight of 45 kg or more,
   wherein a maintenance dose comprising
   about 7 mg to about 11 mg of the bispecific antibody, is administered once monthly to the patient having a body weight from 5 kg to <15 kg, or
   about 18 mg to about 22 mg of the bispecific antibody, is administered once monthly to the patient having a body weight from 15 kg to <45 kg, or
   about 44 mg to about 48 mg of the bispecific antibody, is administered once monthly to the patient having a body weight of 45 kg or more,
   wherein the first maintenance dose is administered one month after administration of the loading dose.
12. The dosage regimen according to embodiment 17 wherein a loading dose of 8.5, 8.6, 8.7, 8.8, 8.9, 9, 9.1, 9.2, 9.3, 9.4 or 9.5 mg of the bispecific antibody is administered to the patient having a body weight from 5 kg to <15 kg, or
   a loading dose of 39.5, 39.6, 39.7, 39.8, 39.9, 40, 40.1, 40.2, 40.3, 40.4 or 40.5 mg of the bispecific antibody is administered to the patient having a body weight from 15 kg to <45 kg, or
   a loading dose of 91.5, 91.6, 91.7, 91.8, 91.9, 92, 92.1, 92.2, 92.3, 92.4, 92.5 mg of the bispecific antibody is administered to the patient having a body weight of 45 kg or more, and wherein
   a maintenance dose of 8.5, 8.6, 8.7, 8.8, 8.9, 9, 9.1, 9.2, 9.3, 9.4 or 9.5 mg of the bispecific antibody is administered once monthly to the patient having a body weight from 5 kg to <15 kg, or
   a maintenance dose of 19.5, 19.6, 19.7, 19.8, 19.9, 20, 20.1, 20.2, 20.3, 20.4 or 20.5 mg of the bispecific antibody is administered once monthly to the patient having a body weight from 15 kg to <45 kg, or
   a maintenance dose of 45.5, 45.6, 45.7, 45.8, 45.9, 46, 46.1, 46.2, 46.3, 46.4 or 46.5 mg of the bispecific antibody is administered once monthly to the patient having a body weight of 45 kg or more.
13. The dosage regimen according to embodiment 17 wherein
   a loading dose of about 9 mg of the bispecific antibody is administered to the patient having a body weight from 5 kg to <15 kg, or
   a loading dose of about 40 mg of the bispecific antibody is administered to the patient having a body weight from 15 kg to <45 kg, or
   a loading dose of about 92 mg of the bispecific antibody is administered to the patient having a body weight of 45 kg or more, and wherein
   a maintenance dose of about 9 mg of the bispecific antibody is administered once monthly to the patient having a body weight from 5 kg to <15 kg, or
   a maintenance dose of about 20 mg of the bispecific antibody is administered once monthly to the patient having a body weight from 15 kg to <45 kg, or
   a maintenance dose of about 46 mg of the bispecific antibody is administered once monthly to the patient having a body weight of 45 kg or more.
14. The dosage regimen according to any of the former embodiments wherein the treatment is a prophylactic treatment.
15. The dosage regimen according to any of the former embodiments wherein a steady state plasma concentration of the bispecific antibody in the range about 2 µg/mL to about 18 µg/mL is provided.
16. The dosage regimen according to any of the former embodiments wherein a steady state plasma concentration of the bispecific antibody in the range about 3 µg/mL to about 9 µg/mL, such as 6.5 µg/mL to 7 µg/mL is provided.
17. The dosage regimen according to any of the former embodiments wherein a steady state plasma concentration of the bispecific antibody of about 6.5 µg/mL is provided.
18. The dosage regimen according to any of the former embodiments wherein the regimen provides an ABR of 0, 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9 or 3 or 4, or an ABR in the range 1-5.
19. The dosage regimen according to any of the former embodiments wherein the bispecific antibody is mab1.
20. A kit a) comprising a pharmaceutical composition comprising the bispecific antibody according to any of embodiments 1-4; and b) instructions for once monthly subcutaneous administration of the pharmaceutical composition for the treatment of haemophilia A with or without inhibitors according to any of the previous embodiments.
21. A method of treatment of haemophilia A with or without inhibitors, comprising subcutaneously administering a bispecific antibody comprising
   an anti-FIX(a) antibody or antigen-binding fragment thereof capable of binding to FIX (SEQ ID NO:1) and/or the activated form thereof (FIXa) comprising a heavy chain and a light chain, and an anti-FX(a) antibody or antigen-binding fragment thereof capable of binding to FX (SEQ ID NO:2) and/or the activated form thereof (FXa), comprising a heavy chain and a light chain,
   wherein the heavy chain of the anti-FIX(a) antibody or antigen-binding fragment thereof comprises CDR1-3 sequences identified by SEQ ID NOs:3, 4 and 5 respectively, and
   the light chain of the anti-FIX(a) antibody or antigen-binding fragment thereof comprises CDR1-3 sequences identified by SEQ ID NOs:8, 9 and 10, respectively, and
   the heavy chain of the anti-FX(a) antibody or antigen-binding fragment thereof comprises CDR1-3 sequences identified by SEQ ID NOs:13, 14 and 15, respectively, and
   the light chain of the anti-FX(a) antibody or antigen-binding fragment thereof comprises CDR1-3 sequences are identified by SEQ ID NOs:18, 19 and 20, respectively;
   in one or more loading dose followed by administration of once monthly maintenance doses,
   wherein the loading dose comprises the same or a higher amount of the bispecific antibody than the maintenance dose, and
   wherein a plasma concentration of the bispecific antibody in the range about 2 µg/mL to about 18 µg/mL is provided.
22. The method of treatment according to embodiment 21 wherein the anti-FIX(a) antibody or antigen-binding fragment thereof comprises
   a heavy chain variable domain identified by SEQ ID NO:6 and a light chain variable domain identified by SEQ ID NO:11, and
   the anti-FX(a) antibody or antigen-binding fragment thereof comprises a heavy chain variable domain identified by SEQ ID NO:16 and a light chain variable domain identified by SEQ ID NO:21.
23. The method of treatment according to embodiment 21 or 22 wherein
   the heavy chain of the anti-FIX(a) antibody comprises SEQ ID NO:7 and the light chain of the anti-FIX(a) antibody comprises SEQ ID NO:12, and
   the heavy chain of the anti-FX(a) antibody comprises SEQ ID NO:17 and the light chain of the anti-FX(a) antibody comprises SEQ ID NO:22.
24. The method of treatment according to any of embodiments 21-23 wherein the bispecific antibody is administered in a pharmaceutical composition comprising the bispecific antibody.
25. The method of treatment according to any of embodiments 21-24 wherein the pharmaceutical composition comprises 1 mg/mL to 100 mg/mL of the bispecific antibody, L-arginine or L-arginine hydrochloride, L-histidine and a surfactant at about pH 5.5 to about pH 7.
26. The method of treatment according to any of embodiments 21-25 wherein the pharmaceutical composition comprises 1 mg/mL to 100 mg/mL of the bispecific antibody, L-arginine or L-arginine hydrochloride, L-histidine and a surfactant at about pH 6.3.
27. The method of treatment according to any of embodiments 21-26 wherein the pharmaceutical composition comprises 1 mg/mL to 100 mg/mL of the bispecific antibody, about 150 mM of L-arginine, about 20 mM of L-histidine and about 0.02% polysorbate 20 or 80 at about pH 6.3.
28. The method of treatment according to any of embodiments 21-27 wherein the pharmaceutical composition comprises 1 mg/mL to 100 mg/mL of the bispecific antibody, about 150 mM of L-arginine hydrochloride, about 20 mM of L-histidine and about 0.02% polysorbate 20 at about pH 6.3.
29. The method of treatment according to any of embodiments 21-28 wherein the pharmaceutical composition comprises 2 mg/mL to 60 mg/mL of mAb1, such as 2 mg/mL, 5 mg/mL, 11.25 mg/mL, 25 mg/mL or 57.5 mg/mL of mAb1, 150 mM of L-arginine hydrochloride, 20 mM of L-histidine and 0.02% polysorbate 20 at pH 6.3.
30. The method of treatment according to any of embodiments 21-29 wherein the bispecific antibody is administered to a human patient.
31. The method of treatment according to any of embodiments 21-30 wherein a loading dose comprising
   about 5 mg to about 15 mg of the bispecific antibody, is administered to a patient having a body weight from 5 kg to <13, or
   about 35 mg to about 45 mg of the bispecific antibody, is administered to a patient having a body weight from 15 kg to <45, or
   about 85 mg to about 95 mg of the bispecific antibody, is administered to a patient having a body weight of 45 kg or more,
   wherein a maintenance dose comprising
   about 7 mg to about 11 mg of the bispecific antibody, is administered once monthly to the patient having a body weight from 5 kg to <15 kg, or
   about 18 mg to about 22 mg of the bispecific antibody, is administered once monthly to the patient having a body weight from 15 kg to <45 kg, or
   about 44 mg to about 48 mg of the bispecific antibody, is administered once monthly to the patient having a body weight of 45 kg or more,
   wherein the first maintenance dose is administered one month after administration of the loading.
32. The method of treatment according to embodiment 31 wherein a loading dose of 8.5, 8.6, 8.7, 8.8, 8.9, 9, 9.1, 9.2, 9.3, 9.4 or 9.5 mg of the bispecific antibody is administered to the patient having a body weight from 5 kg to <15 kg, or
   a loading dose of 39.5, 39.6, 39.7, 39.8, 39.9, 40, 40.1, 40.2, 40.3, 40.4 or 40.5 mg of the bispecific antibody is administered to the patient having a body weight from 15 kg to <45 kg, or
   a loading dose of 91.5, 91.6, 91.7, 91.8, 91.9, 92, 92.1, 92.2, 92.3, 92.4, 92.5 mg of the bispecific antibody is administered to the patient having a body weight of 45 kg or more, and
   wherein
   a maintenance dose of 8.5, 8.6, 8.7, 8.8, 8.9, 9, 9.1, 9.2, 9.3, 9.4 or 9.5 mg of the bispecific antibody is administered once monthly to the patient having a body weight from 5 kg to <15 kg, or
   a maintenance dose of 19.5, 19.6, 19.7, 19.8, 19.9, 20, 20.1, 20.2, 20.3, 20.4 or 20.5 mg of the bispecific antibody is administered once monthly to the patient having a body weight from 15 kg to <45 kg, or
   a maintenance dose of 45.5, 45.6, 45.7, 45.8, 45.9, 46, 46.1, 46.2, 46.3, 46.4 or 46.5 mg of the bispecific antibody is administered once monthly to the patient having a body weight of 45 kg or more.
33. The method of treatment according to embodiment 32 wherein
   a loading dose of about 9 mg of the bispecific antibody is administered to the patient having a body weight from 5 kg to <15 kg, or
   a loading dose of about 40 mg of the bispecific antibody is administered to the patient having a body weight from 15 kg to <45 kg, or
   a loading dose of about 92 mg of the bispecific antibody is administered to the patient having a body weight of 45 kg or more, and wherein
   a maintenance dose about 9 mg of the bispecific antibody is administered once monthly to the patient having a body weight from 5 kg to <15 kg, or
   a maintenance dose of about 20 mg of the bispecific antibody is administered once monthly to the patient having a body weight from 15 kg to <45 kg, or
   a maintenance dose of about 46 mg of the bispecific antibody is administered once monthly to the patient having a body weight of 45 kg or more.
34. The method of treatment according to any of embodiments 21-33 wherein the treatment is prophylactic treatment.
35. The method of treatment according to any of embodiments 21-34 wherein the plasma concentration of mAb1 in the range about 3 µg/mL to about 9 µg/mL, such as 6.5 µg/mL to 7 µg/mL is provided.
36. The method of treatment according to any of embodiments 21-35 wherein the plasma concentration of mAb1 in the range about 6.5 µg/mL is provided.
37. The method of treatment according to any of embodiments 21-36 wherein the plasma concentration is a steady state plasma concentration.
38. The method of treatment according to any of embodiments 21-37 wherein the treatment provides an ABR of 0, 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3 or 4, or an ABR in the range 1-5.
39. The method of treatment according to any of embodiments 21-27 wherein the pharmaceutical composition comprises 1 mg/mL to 100 mg/mL of the bispecific antibody, about 150 mM of L-arginine hydrochloride, about 20 mM of L-histidine and about 0.02% polysorbate 20 at about pH 6.3.
40. The method of treatment according to any of embodiments 21-28 wherein the pharmaceutical composition comprises 2 mg/mL to 60 mg/mL of mAb1, such as 2 mg/mL, 5 mg/mL, 11.25 mg/mL, 25 mg/mL or 57.5 mg/mL of mAb1, 150 mM of L-arginine hydrochloride, 20 mM of L-histidine and 0.02% polysorbate 20 at pH 6.3.
41. The method according to any of embodiments 21-37 wherein the bispecific antibody is mab1.
42. A pharmaceutical composition comprising the bispecific antibody mAb1, about 150 mM of L-arginine hydrochloride, about 20 mM of L-histidine, about 0.02 w/v% polysorbate 20 at about pH 6.3.
43. The pharmaceutical composition according to embodiment 42 wherein said composition comprises about 2 to about 57.5 mg/mL of the bispecific antibody mAb1, about 150 mM of L-arginine hydrochloride, about 20 mM of L-histidine, about 0.02 w/v% polysorbate 20 at about pH 6.3.
44. A kit a) comprising a pharmaceutical composition comprising the bispecific antibody according to any of embodiments 1-19; and b) instructions for once monthly subcutaneous administration of the pharmaceutical composition for the treatment of haemophilia A with or without inhibitors according to any of embodiments 21-41.
45. A kit according to embodiment 20 or 42 wherein the bispecific antibody is mAb1.

In a preferred embodiment the inventors provide a once monthly dosage regimen for use in the treatment of haemophilia A with or without inhibitors, comprising subcutaneously administering a bispecific antibody comprising
an anti-FIX(a) antibody or antigen-binding fragment thereof capable of binding to FIX (SEQ ID NO:1) and/or the activated form thereof (FIXa) comprising a heavy chain and a light chain, and an anti-FX(a) antibody or antigen-binding fragment thereof capable of binding to FX (SEQ ID NO:2) and/or the activated form thereof (FXa), comprising a heavy chain and a light chain,
wherein the heavy chain of the anti-FIX(a) antibody comprises SEQ ID NO:7 and the light chain of the anti-FIX(a) antibody comprises SEQ ID NO:12, and the heavy chain of the anti-FX(a) antibody comprises SEQ ID NO:17 and the light chain of the anti-FX(a) antibody comprises SEQ ID NO:22;
in one loading dose followed by administration of a once monthly maintenance doses;
wherein the bispecific antibody is administered in a pharmaceutical composition comprising the bispecific antibody wherein the pharmaceutical composition comprises 11.25 mg/mL, 25 mg/mL or 57.5 mg/mL of the bispecific antibody, 150 mM of L-arginine hydrochloride, 20 mM of L-histidine and 0.02% polysorbate 20 at about pH 6.3;
wherein
a loading dose of 9 mg of the bispecific antibody is administered to the patient having a body weight from 5 kg to <15 kg, or
a loading dose of 40 mg of the bispecific antibody is administered to the patient having a body weight from 15 kg to <45 kg, or
a loading dose of 92 mg of the bispecific antibody is administered to the patient having a body weight of 45 kg or more, and wherein
a maintenance dose of 9 mg of the bispecific antibody is administered once monthly to the patient having a body weight from 5 kg to <15 kg, or
a maintenance dose of 20 mg of the bispecific antibody is administered once monthly to the patient having a body weight from 15 kg to <45 kg, or
a maintenance dose of 46 mg of the bispecific antibody is administered once monthly to the patient having a body weight of 45 kg or more
wherein a steady state plasma concentration of the bispecific antibody in the range about 2 µg/mL to about 18 µg/mL is provided.

In a preferred embodiment the inventors provide a once monthly dosage regimen for use in the treatment of haemophilia A with or without inhibitors, comprising subcutaneously administering a bispecific antibody comprising
an anti-FIX(a) antibody or antigen-binding fragment thereof capable of binding to FIX (SEQ ID NO:1) and/or the activated form thereof (FIXa) comprising a heavy chain and a light chain, and an anti-FX(a) antibody or antigen-binding fragment thereof capable of binding to FX (SEQ ID NO:2) and/or the activated form thereof (FXa), comprising a heavy chain and a light chain,
wherein the heavy chain of the anti-FIX(a) antibody comprises SEQ ID NO:7 and the light chain of the anti-FIX(a) antibody comprises SEQ ID NO:12, and the heavy chain of the anti-FX(a) antibody comprises SEQ ID NO:17 and the light chain of the anti-FX(a) antibody comprises SEQ ID NO:22;
in one loading dose followed by administration of a once monthly maintenance doses;
wherein the bispecific antibody is administered in a pharmaceutical composition comprising the bispecific antibody wherein the pharmaceutical composition comprises 11.25 mg/mL, 25 mg/mL or 57.5 mg/mL of the bispecific antibody, 150 mM of L-arginine hydrochloride, 20 mM of L-histidine and 0.02 w/v% polysorbate 20 at about pH 6.3;
wherein
a loading dose of 9 mg of the bispecific antibody is administered to the patient having a body weight from 5 kg to <15 kg, or
a loading dose of 40 mg of the bispecific antibody is administered to the patient having a body weight from 15 kg to <45 kg, or
a loading dose of 92 mg of the bispecific antibody is administered to the patient having a body weight of 45 kg or more, and wherein
a maintenance dose of 9 mg of the bispecific antibody is administered once monthly to the patient having a body weight from 5 kg to <15 kg, or
a maintenance dose of 20 mg of the bispecific antibody is administered once monthly to the patient having a body weight from 15 kg to <45 kg, or
a maintenance dose of 46 mg of the bispecific antibody is administered once monthly to the patient having a body weight of 45 kg or more
wherein a steady state plasma concentration of the bispecific antibody in the range about 2 µg/mL to about 18 µg/mL is provided.

A person skilled in the art will understand the various methods for measuring and calculating the pharmacokinetic (for example, but not limited to, Cₘₐₓ, Tₘₐₓ, serum half-life) and pharmacodynamic parameters described herein. Furthermore, the skilled person will understand the various methods for making statistical comparisons (for example, but not limited to, comparisons of change from baseline to post-treatment and/or comparisons among treatment groups) and/or analysis of the pharmacokinetic and pharmacodynamic parameters described herein.

### Examples

### List of Abbreviations

| | |
|---|---|
| CL: | Systemic clearance |
| IIV: | Inter-individual variability |
| Kₐ: | Absorption rate constant |
| MAD: | Multiple Ascending Dose |
| OFV: | Objective function values |
| PwHA: | Patients with haemophilia A |
| PK: | Pharmacokinetics |
| SAD: | Single Ascending Dose |
| SIA: | Sequence-identical analogue |
| Q: | Intercompartmental clearance |
| QW: | Once weekly |
| Q2W: | Once every second week |
| Q4W: | Once every fourth week |
| QM: | Once monthly |
| RSE: | Relative standard error |
| V₂: | Central volume of distribution |
| V₃: | Peripheral volume of distribution |
| F: | Relative bioavailability |

### Example 1: Dose escalation study

The anti-FIX(a)/FX(a) bispecific antibody comprising a first heavy chain comprising SEQ ID NO:7 and a first light chain comprising SEQ ID NO:12 and a second heavy chain comprising SEQ ID NO:17 and a second light chain comprising SEQ ID NO:22 (mAb1) is in development for patients with haemophilia A (PwHA) with or without inhibitors.

FRONTIER1 (EudraCT:2019-000465-20; NCT04204408) aims to investigate the safety, tolerability, pharmacokinetics, pharmacodynamics, and efficacy of single ascending subcutaneous doses of mAb1 (the bispecific antibody) in healthy participants; and multiple ascending doses of mAb1 in PwHA, with or without inhibitors. Additionally, the study aims to provide data for dose-setting in subsequent FRONTIER studies.

**Methods:** In the single ascending dose (SAD) phase, healthy subjects received single ascending doses of mAb1 (targeting plasma concentrations of 0.05 to 3 µg/mL) or placebo. In the multiple ascending dose (MAD) phase, PwHA received multiple ascending doses of mAb1 targeting an average plasma concentration of 1 µg/mL (cohort 1, QW dosing), 3 µg/mL (cohort 2, QW dosing), 9 µg/mL (cohort 3, QW dosing, and cohort 4, Q4W dosing) or 16 µg/mL (cohort 5, QW dosing).

Non-linear mixed-effects modelling was used to analyse mAb1 plasma concentration vs time data from FRONTIER1. Development of the structural base model for mAb1 included one and two-compartment models. The mAb1 plasma concentration-time profiles were best described with a two-compartment model with first-order elimination and absorption rate, parameterised as: absorption rate constant (*kₐ*), systemic clearance (CL), intercompartmental clearance (Q), central volume of distribution (*V₂*), peripheral volume of distribution (*V₃*) and relative bioavailability (*F*, fixed to 1. Random effects were explored with inter-individual variability (IIV, or between-subject variability) on primary parameters of the model and additive and/or proportional residual error. A minimum drop in objective function values (OFV) of 3.84, in addition to model stability and parameter precision, was considered for inclusion of a random effect. The final model included IIV on *CL, kₐ* and *F*, and the residual error was described by a proportional error.

Tables 1-5 list the dosage regimens for MAD cohorts 1-5.

**Table 1: MAD cohort 1 (once weekly dosing) loading- and maintenance dosing**

| Loading dose number 1 and 2 to be administered at week 0 and week 1. | | | | |
|---|---|---|---|---|
| **Loading dose** | | | | |
| **Body weight range (kg)*** | **Dose (mg)** | **mAb1 concentration *(mg*/*mL)*** | **Increments to inject** | **Dose (mL)** |
| **30.0** - **59.9** | 2.7 | 10 | 27 | 0.27 |
| **≥60.0** | 4.9 | 10 | 49 | 0.49 |

| | | | | |
|---|---|---|---|---|
| *based on body weight measured at week 0. | | | | |

Subsequent maintenance doses to be administered from week 2.

| **Maintenance dose** | | | | |
|---|---|---|---|---|
| **Body weight range (kg)*** | **Dose (mg)** | **mAb1 concentration (mg/mL)** | **Increments to inject** | **Dose (mL)** |
| **30.0 - 59.9** | 1.0 | 10 | 10 | 0.10 |
| **≥60.0** | 1.2 | 10 | 12 | 0.12 |

| | | | | |
|---|---|---|---|---|
| *based on latest body weight measured according to Protocol Flowchart. | | | | |

**Table 2: MAD cohort 2 (once weekly dosing) loading- and maintenance dosing**

| Loading dose number 1 and 2 to be administered at week 0 and week 1: | | | | |
|---|---|---|---|---|
| **Loading dose** | | | | |
| **Body weight range (kg)*** | **Dose (mg)** | **mAb1 concentration (mg/mL)** | **Increments to inject** | **Dose (mL)** |
| **30.0 - 59.9** | 15 | 100 | 15 | 0.15 |
| **≥60.0** | 31 | 100 | 31 | 0.31 |

| | | | | |
|---|---|---|---|---|
| *based on body weight measured at week 0. | | | | |

Subsequent maintenance doses to be administered from week 2:

| **Maintenance dose** | | | | |
|---|---|---|---|---|
| **Body weight range (kg)** | **Dose (mg)** | **mAb1 concentration (mg/mL)** | **Increments to inject** | **Dose (mL)** |
| **30.0 - 59.9** | 2.4 | 10 | 24 | 0.24 |
| **≥60.0** | 3.8 | 10 | 38 | 0.38 |

**Table 3: MAD cohort 3 (once weekly dosing) loading- and maintenance dosing**

| Loading dose number 1 and 2 to be administered at week 0 and week 1: | | | | | |
|---|---|---|---|---|---|
| **Loading dose** | | | | | |
| **Body weight range (kg)** | **Dose (mg)** | **mAb1 concentration (mg/mL)** | **Injection no.** | **Increments to inject** | **Dose (mL)** |
| **30.0 - 59.9** | 45 | 100 | 1 | 45 | 0.45 |
| **≥60.0** | 93 | 100 | 1 | 47 | 0.47 |
| | | | 2 | 46 | 0.46 |

Subsequent maintenance doses to be administered from week 2:

| **Maintenance dose** | | | | | |
|---|---|---|---|---|---|
| **Body weight range (kg)** | **Dose (mg)** | **mAb1 concentration (mg/mL)** | **Injection no.** | **Increments to inject** | **Dose (mL)** |
| **30.0 - 59.9** | 10 | 100 | 1 | 10 | 0.10 |
| **≥60.0** | 16 | 100 | 1 | 16 | 0.16 |

**Table 4: MAD cohort 4 (Q4W dosing)**

| One loading dose to be administered at week 0: | | | | | |
|---|---|---|---|---|---|
| **Loading dose** | | | | | |
| **Body weight range (kg)** | **Dose (mg)** | **mAb1 concentration (mg/mL)** | **Injection no.** | **Increments to inject** | **Dose (mL)** |
| **30.0 - 59.9** | 110 | 100 | 1 | 55 | 0.55 |
| | | | 2 | 55 | 0.55 |
| **≥60.0** | 180 | 100 | 1 | 60 | 0.60 |
| | | | 2 | 60 | 0.60 |
| | | | 3 | 60 | 0.60 |

Subsequent doses to be administered from week 4:

| **Maintenance dose** | | | | | |
|---|---|---|---|---|---|
| **Body weight range (kg)** | **Dose (mg)** | **mAb1 concentration (mg/mL)** | **Injection no.** | **Increments to inject** | **Dose (mL)** |
| **30.0 - 59.9** | 41 | 100 | 1 | 41 | 0.41 |
| **≥60.0** | 60 | 100 | 1 | 160 | 0.60 |

**Table 5: MAD cohort 5 (QW dosing)**

| One loading dose to be administered at week 0: | | | | | |
|---|---|---|---|---|---|
| **Loading dose** | | | | | |
| **Body weight range (kg)** | **Dose (mg)** | **mAb1 concentration (mg/mL)** | **Injection no.** | **Increments to inject** | **Dose (mL)** |
| **30.0** - **59.9** | 83 | 100 | 1 | 83 | 0.82 |
| **≥60.0** | 150 | 100 | 1 | 75 | 0.75 |
| | | | 2 | 75 | 0.75 |

Subsequent maintenance doses to be administered from week 2:

| **Maintenance dose** | | | | | |
|---|---|---|---|---|---|
| **Body weight range (kg)** | **Dose (mg)** | **mAb1 concentration (mg/mL)** | **Injection no.** | **Increments to inject** | **Dose (mL)** |
| **30.0 - 59.9** | 24 | 100 | 1 | 24 | 0.24 |
| **≥60.0** | 35 | 100 | 1 | 35 | 0.35 |

**Results**: mAb1 was well tolerated following both single and multiple dosing, and no related thromboembolic events or serious adverse events were reported. No occurrences of anti-mAb1 antibodies were reported. Data from the SAD section provides an estimated T_{1/2} of 30.4 days, and Tₘₐₓ of 9.1 days. Highest mAb1 concentration observed in MAD cohort 5 was 18 µg/mL, which was evaluated as safe and effective (Figure 2).

Population PK model analysis of the FRONTIER1 data suggested that the PK of mAb1 is best described by a two-compartment PK model with first-order absorption and elimination. Main covariate factors influencing mAb1 concentration were baseline body weight and drug product strength. The parameter estimates from the population PK model analysis are collected in Table 6 and fit of the model to the FRONTIER1 MAD data is presented in Figure 3.

**Table 6: Parameter estimates of the population PK model**

| **Parameter** | **Estimate** | **RSE%** | **Description** |
|---|---|---|---|
| **CL/F (L/day)** | 0.201 | 4 | Apparent clearance |
| **V2/F (L)** | 5.81 | 12 | Apparent volume of distribution, central |
| **V3/F (L/day)** | 2.58 | 23 | Apparent volume of distribution, peripheral |
| **Q/F (L)** | 10.8 | 22 | Apparent inter-compartment clearance |
| **KA (1/day)** | 0.304 | 6 | Absorption rate constant |
| **cov CL_BW** | 0.817 | 19 | Covariate, body weight on clearance, exponent |
| **cov V_BW** | 0.968 | 12 | Covariate, body weight on central V, exponent |
| **cov F_DP SAD** | 0.747 | 6 | Covariate SAD, relative bioavailability change, DP 100 mg/mL |
| **cov F_DP MAD** | 0.831 | 5 | Covariate MAD, relative bioavailability change, DP 100 mg/mL |
| **IIV_CL (%)** | 28.4 | 16 | Inter-individual variability on CL |
| **IIV_KA (%)** | 64.6 | 8 | Inter-individual variability on KA |
| **IIV_F (%)** | 22.0 | 11 | Inter-individual variability on F |
| **PropRE** | 0.154 | 6 | Proportional residual error |

| | | | |
|---|---|---|---|
| RSE: Relative standard error | | | |

During the 12-week observation period, 15 treated bleeds were reported in 8 patients, of which 13 bleeds (9 traumatic) were observed in 6 patients from the lowest dose cohort. The 2 bleeds in patients from cohorts 2 and 3 were traumatic, thus neither treated joint nor spontaneous bleeds were observed beyond cohort 1 (Table 7).

**Conclusion:** The pharmacokinetic properties support a once monthly dosing approach. The population PK model adequately described the median trend and variability in data with main covariates being baseline body weight and drug product strength. mAb1 was well tolerated and no occurrences of anti-mAb1 antibodies were reported. FRONTIER1 provides encouraging data supporting further clinical development using methods of administration / dosage regimens as described herein which were derived by taking influence of body weight and drug product strength into account.

**Table 7: Treated bleeds during the 12 weeks of treatment of PwHA with multiple ascending doses of mAb1**

| | | **Cohort 1** | **Cohort 2** | **Cohort 3** | **Cohort 4** | **Cohort 5** |
|---|---|---|---|---|---|---|
| **Average planned plasma concentration (dosing regimen)** | | 1 µg/mL (QW dosing) | 3 µg/mL (QW dosing) | 9 µg/mL (QW dosing) | 9 µg/mL (Q4W dosing) | 18 µg/mL (QW dosing) |
| **Number of subjects** | | 7 | 9 | 8 | 8 | 10 |
| **Total exposure time (years)** | | 1.50 | 2.05 | 1.80 | 1.73 | 2.31 |
| **Number of bleeds** | | 13 | 1 | 1 | - | 11 |
| **Number of subjects with bleeds, N (%)** | | 6 (85.7) | 1 (11.1) | 1 (12.5) | - | 3 (30) |
| **Number of subjects with zero bleeds, N (%)** | | 1 (14.3) | 8 (88.9) | 7 (87.5) | 8 (100.0) | 7 (70) |
| **Severe bleeds, N (%)** | | - | - | - | - | - |

| **Cause of bleed, N (%)** | | | | | | |
|---|---|---|---|---|---|---|
| | Spontaneous | 4 (30.8) | - | - | - | 10 (91) |
| | Traumatic | 9 (69.2) | 1 (100.0) | 1 (100.0) | - | 1 (9) |

| **Site of bleed, N (%)** | | | | | | |
|---|---|---|---|---|---|---|
| | **Joint** | 11 (84.6) | - | - | - | 10 (91) |
| | Muscle | 2 (15.4) | - | 1 (100.0) | - | 1 (9) |
| | Other | - | 1 (100.0) | - | - | - |

### Example 2: Thrombin generation following administration of mAb1

Factor VIII (FVIII) replacement is the standard of care for patients with haemophilia A (HA). mAb1 is a bispecific antibody capable of combining Factor IX(a) and FX(a) with enhanced haemostatic properties *in vitro* and in HA mouse models, compared with emicizumab. FRONTIER1 (NCT04204408) is a phase 1/2 study investigating the safety, tolerability, pharmacokinetics, and pharmacodynamics of subcutaneously administered mAb1 in healthy volunteers and patients with severe HA, independent of FVIII inhibitor status.

Peak thrombin generation, and laboratory markers in response to mAb1 or emicizumab were analysed.

**Methods:** The phase 2 part of FRONTIER1 is open-label, with mAb1 administered subcutaneously over 12 weeks, across five multiple ascending dose cohorts, targeting an average plasma concentration of 1-18 µg/mL through dosing weekly (cohorts 1-3 and 5) or every fourth week (cohort 4); cohorts 3/4 targeted the same plasma concentration. An additional exploratory cohort of subjects treated with emicizumab was included for comparison.

**Results:** 42 subjects on mAb1 (cohorts 1 [n=7], 2 [n=9], 3 [n=8], 4 [n=8] and 5 [n=10]) and 10 on emicizumab were included**.** Peak thrombin levels increased with mAb1 dose. In an *in vitro* experiment where pre-dose FVIII-neutralised plasma samples were spiked with mAb1 or emicizumab, maximal peak height was reached at lower plasma concentrations than with emicizumab, indicating higher potency for mAb1 (see Figure 4). Mean peak thrombin levels were comparable between patients on emicizumab and mAb1 in cohort 2. No dose-dependent changes in D-dimer, fibrinogen, platelets, or FIXa/FX antigen levels were observed; most values remained within normal range. A dose-dependent increase in prothrombin fragments 1 and 2 was observed for patients on mAb1 and emicizumab (relative change [%]: 26.08, 93.99, 323.65, 606.41 and 315.62 in cohorts 1-5, respectively, and 85.04 for emicizumab), with stabilisation at steady state.

**Conclusions:** A dose-dependent increase in thrombin generation was observed in mAb1 patients, reaching higher peak thrombin levels than in emicizumab patients. Laboratory parameters showed no safety signals.

### Example 3: Extrapolating results from Example 1 and 2 to novel dosage regimens for different weight bands

Based on the specific novel learnings from the FRONTIER1 clinical trials such as those included in Examples 1 and 2, the present inventors have devised the methods of **administration** and in particular the specific dosage regimens (including selection of weight bands/groups) as disclosed herein by carefully analysing and using the novel data obtained in said trials, including patient response and observed properties of the bispecific antibody mAb1 (such as dose, T_{½}, Tₘₐₓ and plasma concentration. The inventors have for example arrived at a therapeutic steady state plasma concentration in the range about 2 µg/mL to about 18 µg/mL, preferably 3 to 9 µg/ml such as 5, 5.5, 6, 6.5 or 7 µg/ml and developed the necessary dosage regimens to reach this steady state plasma concentration for particular weight bands by expanding the number of weight bands from two to three such to cover body weights from 5 kg and consequently also of paediatric patients.

Using the population PK model described in Example 1, the inventors have derived dosing regimens that rapidly establish a steady state mAb1 plasma concentration within the therapeutic range (2 to 18 µg/mL) with one loading dose, followed by a maintenance dose for QW, Q2W and QM dosing frequencies for typical body weights within a haemophilia population.

Examples of said dosing regimens and weight bands are shown in Table 8.

**Table 8: Examples of maintenance- and loading doses and weight bands for mAb1**

| **Weight band (kg)** | **Dose QM (mg)** | |
|---|---|---|
| | Maintenance dose | Loading dose |
| **5 - <15** | 9 | 9 |
| **15 - <45** | 20 | 40 |
| **≥45** | 46 | 92 |

| | | |
|---|---|---|
| QM = once monthly. | | |

Examples of said mAb1 PK profiles for typical subjects within each weight band and dosing frequency are shown in Figure 5 (C2 = cohort 2, C_{avg} = average plasma concentration, Cₘₐₓ = maximum plasma concentration).

### Example 4: Stability of mAb1 compositions

The pharmaceutical compositions analysed in the present example comprise 1-100 mg/mL of the bispecific antibody mAb1, 150 mM of L-arginine hydrochloride, 20 mM of L-histidine, 0.02 w/v% polysorbate 20 at apx. pH 6.3.

Stability was assessed based on the following key parameters: Appearance, High Molecular Weight Proteins *(*HMWP), Monomer and Purity. Based on the stability results given in Table 9 to Table 22 no or only a minor change in trend is observed for the key parameters during storage at long-term storage conditions (5°C ± 3°C) and at accelerated storage conditions (25°C ± 2°C) related to chemical stability (HMWP, Monomer and Purity) and physical stability (Appearance).

In summary, the results show that the compositions are chemically and physically stable over time at 5°C and 25°C.

### Analytical procedures

### 1. Compendial analytical procedures

The following compendial analytical procedures are employed for the control of the drug products.

### Appearance

The appearance of the drug products is determined by visual inspection according to Ph. Eur., and JP.

### pH

pH is measured by a potentiometric determination performed according to Ph. Eur., USP, and JP.

### 2. Non-compendial analytical procedures

The following non-compendial analytical procedures are employed for the control of drug products.

### 2.1 Content by SE-HPLC

Content is determined by SE-HPLC using isocratic elution on a size-exclusion column and subsequent UV detection. The Content is calculated using the areas of the high molecular weight proteins (HMWP) peak and the monomer peak relative to the areas of the HMWP peak and the monomer peak of the reference material with a known content concentration and expressed in mg/ml.

### 2.2 Monomer by SE-HPLC

The content of monomer is determined by SE-HPLC using isocratic elution on a size-exclusion column and subsequent UV detection. The monomer peak area relative to the total area is calculated and expressed in percent.

### 2.3 High Molecular Weight Proteins (HMWP) by SE-HPLC

The content of HMWP is determined by SE-HPLC using isocratic elution on a size-exclusion column and subsequent UV detection. The HMWP peak area relative to the total area is calculated and expressed in percent.

### 2.4 Purity by CE-SDS

Purity is defined as the main peak area by capillary electrophoresis in presence of sodium dodecyl sulphate (CE-SDS). The Purity is determined by CE-SDS under nonreducing conditions and with UV detection. The Purity is calculated as the main peak area relative to the total area and expressed in percent.

### Stability data for compositions comprising mAb1 in a concentration range of 1-100 mg/ml

Stability of compositions comprising 1, 2, 5, 11.25, 57.5 and 100 mg/mL mAb1 was followed at long-term storage conditions (5°C ± 3°C) and at accelerated storage conditions (25°C ± 2°C).

The results for compositions comprising mAb1 in the range of 1 mg/ml to 100 mg/ml show comparable stability. The compositions are chemically and physically stable.

### Stability data for 1 mg/ml mAb1

The stability data of compositions comprising 1 mg/ml mAb1 are reported in Table 9 for long-term storage conditions and in Table 10 for accelerated storage conditions.

**Table 9: Stability data for 1 mg/ml mAb1 at 5°C±3°C**

| **Test parameter** | **Timepoint (months) at 5°C±3°C** | | | | | |
|---|---|---|---|---|---|---|
| | 0 | 3 | 6 | 9 | 12 | 18 |
| Appearance | Complies¹ | Complies¹ | Complies¹ | Complies¹ | Complies¹ | Complies¹ |
| pH | 6.4 | 6.4 | 6.3 | 6.3 | 6.3 | 6.3 |
| Content (mg/ml) | 1.0 | 1.0 | 1.0 | 1.0 | 1.0 | 1.0 |
| HMWP (%) | 0.5 | 0.6 | 0.6 | 0.5 | 0.6 | 0.6 |
| Monomer (%) | 99.4 | 99.2 | 99.3 | 99.1 | 99.4 | 99.3 |
| Purity (%) | 97.6 | 97.5 | 97.5 | 96.8 | 97.1 | 96.7 |

| | | | | | | |
|---|---|---|---|---|---|---|
| ¹ Complies means: A clear or almost clear, colourless or almost colourless liquid, essentially free of particles | | | | | | |

**Table 10: Stability data for 1 mg/ml mAb1 at 25°C±2°C**

| **Test parameter** | **Timepoint (months) at 25°C±2°C** | | | |
|---|---|---|---|---|
| | 0 | 1 | 3 | 6 |
| Appearance | Complies¹ | Complies¹ | Complies¹ | Complies¹ |
| pH | 6.4 | 6.3 | 6.3 | 6.3 |
| Content (mg/ml) | 1.0 | 1.1 | 1.0 | 1.0 |
| HMWP (%) | 0.5 | 0.6 | 0.6 | 0.6 |
| Monomer (%) | 99.4 | 99.2 | 99.2 | 99.2 |
| Purity (%) | 97.6 | 96.0 | 95.7 | 94.1 |

| | | | | |
|---|---|---|---|---|
| ¹ Complies means: A clear or almost clear, colourless or almost colourless liquid, essentially free of particles | | | | |

### Stability data for 2.0 mg/ml mAb1

The stability data for 2.0 mg/ml mAb1 are reported in Table 11 for long-term storage conditions, and in Table 12 for accelerated storage conditions.

**Table 11: Stability data for 2.0 mg/ml mAb1 at 5°C±3°C**

| **Test parameter** | **Timepoint (months) at 5°C±3°C** | |
|---|---|---|
| | 0 | 3 |
| Appearance | Complies¹ | Complies¹ |
| pH | 6.3 | 6.3 |
| Content (mg/ml) | 1.99 | 2.00 |
| HMWP (%) | 0.8 | 0.8 |
| Monomer (%) | 99.2 | 99.1 |
| Purity (%) | 97.4 | 97.5 |

| | | |
|---|---|---|
| ¹ Complies means: A clear or almost clear, colourless or almost colourless liquid, essentially free of particles | | |

**Table 12: Stability data for 2.0 mg/ml mAb1 at 25°C±2°C**

| **Test parameter** | **Timepoint (months) at 25°C±2°C** | |
|---|---|---|
| | 0 | 1 |
| Appearance | Complies¹ | Complies¹ |
| pH | 6.3 | 6.3 |
| Content (mg/ml) | 2.00 | 2.00 |
| HMWP (%) | 0.8 | 0.8 |
| Monomer (%) | 99.1 | 99.1 |
| Purity (%) | 97.5 | 97.8 |

| | | |
|---|---|---|
| ¹ Complies means: A clear or almost clear, colourless or almost colourless liquid, essentially free of particles | | |

### Stability data for 5.0 mg/ml mAb1

The stability data for 5.0 mg/ml mAb1 are reported in Table 13 for long-term storage conditions, and in Table 14 for accelerated storage conditions.

**Table 13: Stability data for 5.0 mg/ml mAb1 at 5°C±3°C**

| **Test parameter** | **Timepoint (months) at 5°C±3°C** | |
|---|---|---|
| | 0 | 3 |
| Appearance | Complies¹ | Complies¹ |
| pH | 6.3 | 6.3 |
| Content (mg/ml) | 4.90 | 4.91 |
| HMWP (%) | 0.7 | 0.7 |
| Monomer (%) | 99.2 | 99.2 |
| Purity (%) | 97.0 | 97.9 |

| | | |
|---|---|---|
| ¹ Complies means: A clear or almost clear, colourless or almost colourless liquid, essentially free of particles | | |

**Table 14: Stability data for 5.0 mg/ml mAb1 at 25°C±2°C**

| **Test parameter** | **Timepoint (months) at 25°C±2°C** | |
|---|---|---|
| | 0 | 1 |
| Appearance | Complies¹ | Complies¹ |
| pH | 6.3 | 6.3 |
| Content (mg/ml) | 4.91 | 4.90 |
| HMWP (%) | 0.7 | 0.8 |
| Monomer (%) | 99.2 | 99.1 |
| Purity (%) | 97.9 | 97.6 |

| | | |
|---|---|---|
| ¹ Complies means: A clear or almost clear, colourless or almost colourless liquid, essentially free of particles | | |

### Stability data for 11.3 mg/ml mAb1

The stability data for 11.3 mg/ml mAb1 are reported in Table 15 for long-term storage conditions, and in Table 16 for accelerated storage conditions.

**Table 15: Stability data for 11.3 mg/ml mAb1 at 5°C±3°C**

| **Test parameter** | **Timepoint (months) at 5°C±3°C** | |
|---|---|---|
| | 0 | 3 |
| Appearance | Complies¹ | Complies¹ |
| pH | 6.3 | 6.3 |
| Content (mg/ml) | 11.2 | 11.2 |
| HMWP (%) | 0.8 | 0.9 |
| Monomer (%) | 99.1 | 99.0 |
| Purity (%) | 97.3 | 96.5 |

| | | |
|---|---|---|
| ¹ Complies means: A clear or almost clear, colourless or almost colourless liquid, essentially free of particles | | |

**Table 16: Stability data for 11.3 mg/ml mAb1 at 25°C±2°C**

| **Test parameter** | **Timepoint (months) at 25°C±2°C** | |
|---|---|---|
| | 0 | 1 |
| Appearance | Complies¹ | Complies¹ |
| pH | 6.3 | 6.3 |
| Content (mg/ml) | 11.2 | 11.2 |
| HMWP (%) | 0.9 | 0.9 |
| Monomer (%) | 99.0 | 99.0 |
| Purity (%) | 96.5 | 96.2 |

| | | |
|---|---|---|
| ¹ Complies means: A clear or almost clear, colourless or almost colourless liquid, essentially free of particles | | |

### Stability data for 25.0 mg/ml mAb1

The stability data for 25.0 mg/ml mAb1 are reported in Table 17 for long-term storage conditions, and in Table 18 for accelerated storage conditions.

**Table 17: Stability data for 25.0 mg/ml mAb1 at 5°C±3°C**

| **Test parameter** | **Timepoint (months) at 5°C±3°C** | |
|---|---|---|
| | 0 | 3 |
| Appearance | Complies¹ | Complies¹ |
| pH | 6.2 | 6.3 |
| Content (mg/ml) | 24.6 | 24.6 |
| HMWP (%) | 0.9 | 0.9 |
| Monomer (%) | 99.0 | 99.0 |
| Purity (%) | 97.3 | 97.4 |

| | | |
|---|---|---|
| ¹ Complies means: A clear or almost clear, colourless or almost colourless liquid, essentially free of particles | | |

**Table 18: Stability data for 25.0 mg/ml mAb1 at 25°C±2°C**

| **Test parameter** | **Timepoint (months) at 25°C±2°C** | |
|---|---|---|
| | 0 | 1 |
| Appearance | Complies¹ | Complies¹ |
| pH | 6.2 | 6.3 |
| Content (mg/ml) | 24.6 | 24.7 |
| HMWP (%) | 0.9 | 1.0 |
| Monomer (%) | 99.0 | 98.9 |
| Purity (%) | 97.3 | 96.9 |

| | | |
|---|---|---|
| ¹ Complies means: A clear or almost clear, colourless or almost colourless liquid, essentially free of particles | | |

### Stability data for 57.5 mg/ml mAb1

The stability data for 57.5 mg/ml mAb1 are reported in Table 19 for long-term storage conditions, and in Table 20 for accelerated storage conditions.

**Table 19: Stability data for 57.5 mg/ml mAb1 at 5°C±3°C**

| **Test parameter** | **Timepoint (months) at 5°C±3°C** | |
|---|---|---|
| | **0** | **3** |
| Appearance | Complies¹ | Complies¹ |
| pH | 6.3 | 6.3 |
| Content (mg/ml) | 56.7 | 55.8 |
| HMWP (%) | 0.9 | 1.0 |
| Monomer (%) | 99.1 | 98.9 |
| Purity (%) | 97.3 | 97.6 |

| | | |
|---|---|---|
| ¹ Complies means: A clear or almost clear, colourless or almost colourless liquid, essentially free of particles | | |

**Table 20: Stability data for 57.5 mg/ml mAb1 at 25°C±2°C**

| **Test parameter** | **Timepoint (months) at 25°C±2°C** | |
|---|---|---|
| | 0 | 1 |
| Appearance | Complies¹ | Complies¹ |
| pH | 6.3 | 6.3 |
| Content (mg/ml) | 55.8 | 56.5 |
| HMWP (%) | 1.0 | 1.1 |
| Monomer (%) | 98.9 | 98.8 |
| Purity (%) | 97.6 | 96.9 |

| | | |
|---|---|---|
| ¹ Complies means: A clear or almost clear, colourless or almost colourless liquid, essentially free of particles | | |

### Stability data for 100 mg/ml mAb1

The stability data for 100 mg/ml mAb1 are reported in Table 21 for long-term storage conditions and in Table 22 for accelerated storage conditions.

**Table 21: Stability data for 100 mg/ml mAb1 at 5°C±3°C**

| **Test parameter** | **Timepoint (months) at 5°C±3°C** | | | | | |
|---|---|---|---|---|---|---|
| | 0 | 3 | 6 | 9 | 12 | 18 |
| Appearance | Complies¹ | Complies¹ | Complies¹ | Complies¹ | Complies¹ | Complies¹ |
| pH | 6.3 | 6.3 | 6.3 | 6.3 | 6.3 | 6.2 |
| Content (mg/ml) | 99.9 | 99.3 | 98.7 | 100.8 | 100.6 | 101.9 |
| HMWP (%) | 0.6 | 0.8 | 0.9 | 0.9 | 1.0 | 1.0 |
| Monomer (%) | 99.3 | 99.0 | 99.0 | 98.7 | 98.9 | 98.8 |
| Purity (%) | 97.7 | 96.0 | 97.5 | 97.1 | 97.1 | 97.1 |

| | | | | | | |
|---|---|---|---|---|---|---|
| ¹ Complies means: A clear or almost clear, colourless or almost colourless liquid, essentially free of particles | | | | | | |

**Table 22: Stability data for 100 mg/ml mAb1at 25°C±2°C**

| **Test parameter** | **Timepoint (months) at 25°C±2°C** | | | |
|---|---|---|---|---|
| | 0 | 1 | 3 | 6 |
| Appearance | Complies' | Complies¹ | Complies¹ | Complies¹ |
| pH | 6.3 | 6.3 | 6.3 | 6.3 |
| Content (mg/ml) | 99.9 | 99.9 | 99.1 | 98.6 |
| HMWP (%) | 0.6 | 0.9 | 1.1 | 1.4 |
| Monomer (%) | 99.3 | 98.9 | 98.7 | 98.4 |
| Purity (%) | 97.7 | 97.1 | 95.1 | 93.7 |

| | | | | |
|---|---|---|---|---|
| ¹ Complies means: A clear or almost clear, colourless or almost colourless liquid, essentially free of particles | | | | |

While certain features of the invention have been illustrated and described herein, many modifications, substitutions, changes, and equivalents will now occur to those of ordinary skill in the art. It is, therefore, to be understood that the appended claims are intended to cover all such modifications and changes as fall within the true spirit of the invention.

## Claims

1. A bispecific antibody for use in the treatment of haemophilia A with or without inhibitors, wherein the bispecific antibody comprises
an anti-FIX(a) antibody or antigen-binding fragment thereof capable of binding to FIX (SEQ ID NO:1) and/or the activated form thereof (FIXa) comprising a heavy chain and a light chain, and
an anti-FX(a) antibody or antigen-binding fragment thereof capable of binding to FX (SEQ ID NO:2) and/or the activated form thereof (FXa), comprising a heavy chain and a light chain,
wherein
the heavy chain of the anti-FIX(a) antibody or antigen-binding fragment thereof comprises CDR1-3 sequences identified by SEQ ID NOs:3, 4 and 5, respectively, and
the light chain of the anti-FIX(a) antibody or antigen-binding fragment thereof comprises CDR1-3 sequences identified by SEQ ID NOs:8, 9 and 10, respectively, and
the heavy chain of the anti-FX(a) antibody or antigen-binding fragment thereof comprises CDR1-3 sequences identified by SEQ ID NOs:13, 14 and 15, respectively, and
the light chain of the anti-FX(a) antibody or antigen-binding fragment thereof comprises CDR1-3 sequences identified by SEQ ID NOs:18, 19 and 20, respectively, and
wherein the bispecific antibody is to be administered subcutaneously to a human patient in a composition comprising the bispecific antibody,
wherein a loading dose comprising
- about 9 mg of the bispecific antibody, is administered to a patient having a body weight from 5 kg to <15, or
- about 40 mg of the bispecific antibody, is administered to a patient having a body weight from 15 kg to <45, or
- about 92 mg of the bispecific antibody, is administered to a patient having a body weight of 45 kg or more,
wherein a maintenance dose comprising
- about 9 mg of the bispecific antibody, is administered once monthly to the patient having a body weight from 5 kg to <15 kg, or
- about 20 mg of the bispecific antibody, is administered once monthly to the patient having a body weight from 15 kg to <45 kg, or
- about 46 mg of the bispecific antibody, is administered once monthly to the patient having a body weight of 45 kg or more,
wherein the first maintenance dose is administered one month after administration of the loading dose; and
wherein a steady state plasma concentration of the bispecific antibody in the range about 2 µg/mL to about 18 µg/mL, such as 3 to 9 µg/mL, such as 6.5 µg/mL is provided.

2. The bispecific antibody for use in the treatment of haemophilia A with or without inhibitors according to claim 1
wherein the anti-FIX(a) antibody or antigen-binding fragment thereof comprises a heavy chain variable domain identified by SEQ ID NO:6 and a light chain variable domain identified by SEQ ID NO:11, and
wherein the anti-FX(a) antibody or antigen-binding fragment thereof comprises a heavy chain variable domain identified by SEQ ID NO:16 and a light chain variable domain identified by SEQ ID NO:21.

3. The bispecific antibody for use in the treatment of haemophilia A with or without inhibitors according to claim 2 wherein
the heavy chain of the anti-FIX(a) antibody comprises SEQ ID NO:7 and the light chain of the anti-FIX(a) antibody comprises SEQ ID NO:12, and
the heavy chain of the anti-FX(a) antibody comprises SEQ ID NO:17 and the light chain of the anti-FX(a) antibody comprises SEQ ID NO:22.

4. The bispecific antibody for use in the treatment of haemophilia A with or without inhibitors according to any of the previous claims wherein the bispecific antibody is mAb1.

5. The bispecific antibody for use in the treatment of haemophilia A with or without inhibitors according to any of the previous claims wherein a loading dose comprising
- 9 mg of the bispecific antibody, is administered to a patient having a body weight from 5 kg to <15, or
- 40 mg of the bispecific antibody, is administered to a patient having a body weight from 15 kg to <45, or
- 92 mg of the bispecific antibody, is administered to a patient having a body weight of 45 kg or more,
wherein a maintenance dose comprising
- 9 mg of the bispecific antibody, is administered once monthly to the patient having a body weight from 5 kg to <15 kg, or
- 20 mg of the bispecific antibody, is administered once monthly to the patient having a body weight from 15 kg to <45 kg, or
- 46 mg of the bispecific antibody, is administered once monthly to the patient having a body weight of 45 kg or more,
wherein
a steady state plasma concentration of the bispecific antibody in the range 3 to 9 µg/mL, such as 6.5 µg/mL is provided.

6. A bispecific antibody for use in the treatment of haemophilia A with or without inhibitors, wherein the bispecific antibody is mAb1, and
wherein mAb1 is to be administered subcutaneously to a human patient in a composition comprising mAb1,
wherein a loading dose comprising
- 9 mg of mAb1, is administered to a patient having a body weight from 5 kg to <15,
or
- 40 mg of mAb1, is administered to a patient having a body weight from 15 kg to <45,
or
- 92 mg of mAb1, is administered to a patient having a body weight of 45 kg or more,
wherein a maintenance dose comprising
- 9 mg of mAb1, is administered once monthly to the patient having a body weight from 5 kg to <15 kg, or
- 20 mg of mAb1, is administered once monthly to the patient having a body weight from 15 kg to <45 kg, or
- 46 mg of mAb1, is administered once monthly to the patient having a body weight of 45 kg or more,
wherein the first maintenance dose is administered one month after administration of the loading dose; and
wherein a steady state plasma concentration of mAb1 in the range 3 to 9 µg/mL, such as 6.5 µg/mL is provided.

7. The bispecific antibody for use in the treatment of haemophilia A with or without inhibitors according to any of the previous claims wherein the composition comprises about 150 mM of L-arginine hydrochloride or L-arginine, about 20 mM of L-histidine and a surfactant such as polysorbate 20 or polysorbate 80 at about pH 6.3.

8. The bispecific antibody for use in the treatment of haemophilia A with or without inhibitors according to any of the previous claims wherein the composition comprises about 150 mM of L-arginine hydrochloride or L-arginine, about 20 mM of L-histidine and about 0.02 w/v% polysorbate 20 or polysorbate 80 at about pH 6.3.

9. The bispecific antibody for use in the treatment of haemophilia A with or without inhibitors according to any of the previous claims wherein the composition comprises about 150 mM of L-arginine hydrochloride, about 20 mM of L-histidine and about 0.02 w/v% polysorbate 20 at about pH 6.3.

10. The bispecific antibody for use in the treatment of haemophilia A with or without inhibitors according to any of the previous claims wherein the composition comprises 150 mM of L-arginine hydrochloride, 20 mM of L-histidine and 0.02 w/v% polysorbate 20 at pH 6.3.

11. The bispecific antibody for use in the treatment of haemophilia A with or without inhibitors according to any of the previous claims wherein the composition comprises about 2, about 5, about 11.25, about 25 or about 57.5 mg/ml of the bispecific antibody.

12. The bispecific antibody for use in the treatment of haemophilia A with or without inhibitors according to any of the previous claims wherein the treatment provides an annualized bleeding rate of 0, 1, 2, 3 or 4.

13. A pharmaceutical composition comprising the bispecific antibody mAb1, about 150 mM of L-arginine hydrochloride, about 20 mM of L-histidine, about 0.02 w/v% polysorbate 20 at about pH 6.3.

14. The pharmaceutical composition according to claim 13 wherein said composition comprises about 2 to about 57.5 mg/mL of the bispecific antibody mAb1, about 150 mM of L-arginine hydrochloride, about 20 mM of L-histidine, about 0.02 w/v% polysorbate 20 at about pH 6.3.

15. A kit comprising a) a pharmaceutical composition comprising the bispecific antibody according to any of claims 1-4; and b) instructions for once monthly subcutaneous administration of the pharmaceutical composition for the treatment of haemophilia A with or without inhibitors according to any of the previous claims.
